# EUROPEAN PATENT SPECIFICATION

(11) **EP 0 653 067 B1**
(45) Date of publication and mention of the grant of the patent: **22.01.2003**
(21) Application number: 93918536.9
(22) Date of filing: 30.07.1993
(51) Int. Cl.: G01N 33/68, C07K 2/00

(54) **METHOD AND KIT FOR PYRIDINIUM CROSSLINK ASSAY**
VERFAHREN UND TESTSATZ FÜR PYRIDINIUM-CROSSLINK-ASSAY
PROCEDE ET MATERIEL DE RETICULATION EN PRESENCE D'IONS PYRIDIUM

(30) Priority: 31.07.1992 US 922906; 17.12.1992 US 992888; 26.03.1993 US 37571; 26.03.1993 US 37602
(43) Date of publication of application: 17.05.1995
(73) Proprietor: METRA BIOSYSTEMS, INC., Mountain View, California 94043-3911 (US)
(72) Inventor: CERELLI, Mary, J., Burlingame, CA 94010 (US); DANILOFF, George, Y., Mountain View, CA 94040 (US); KUNG, Viola, T., Menlo Park, CA 94025 (US); ZUK, Robert, F., Burlingame, CA 94010 (US); JU, Hsin-Shan, Julia, Cupertino, CA 95014 (US); EVANS, Brian, Jeffrey, Santa Clara, CA 95054 (US); HE, Pingren, Burlingame, CA 94010 (US)
(74) Representative: Hallybone, Huw George
(86) International application number: US9307203
(87) International publication number: WO94003814

(56) References cited:
- WO-A-89/04491
- WO-A-89/12824
- CALCIFIED TISSUE INTERNATIONAL, vol.52, no.SUP1, June 1993, BERLIN, DE; NEW YORK, NY, US page S92 V.T. KUNG ET AL. 'A Monoclonal Immunoassay for Collagen Crosslinks' XXIIIrd European Symposium on Calcified Tissues; HEIDELBERG, DE, April 25-29, 1993
- BIOCHIMICA ET BIOPHYSICA ACTA, vol.914, 1987, AMSTERDAM, NL pages 233 - 239 SIMON P. ROBINS AND ALEXANDER DUNCAN 'Pyridinium crosslinks of bone collagen and their location in peptides isolated from rat femur' cited in the application
- ANNALS OF THE RHEUMATIC DISEASES, vol.45, no.12, December 1986, LONDON, GB pages 969 - 973 SIMON P. ROBINS ET AL. 'Measurement of the cross linking compound, pyridinoline, in urine as an index of collagen degradation in joint disease' cited in the application
- JOURNAL OF BONE AND MINERAL RESEARCH, vol.7, no.11, November 1992, NEW YORK, NY, US pages 1251 - 1258 DENNIS A. HANSON ET AL. 'A Specific Immunoassay for Monitoring Human Bone Resorption: Quantitation of Type I Collagen Cross-linked N-Telopeptides in Urine'
- ANNALS OF RHEUMATIC DISEASES, vol.48, no.8, August 1989, LONDON, BG pages 641 - 644 D.BLACK ET AL. 'Urinary excretion of the hydroxypyridinium cross links of collagen in patients with rheumatoid arthritis' cited in the application

## Description

### 1. Field of the Invention

The present invention relates to a method for determining native free pyridinium crosslink levels in human urine samples, and to an antibody reagent and kit for use in the method.

### 2. References

Black, D., *et al., Anal. Biochem.* 169:197-203 (1988).
Black, D., *et al., Annals of Rheumatic Diseases* 48:641-644 (1989).
Brown, J.P., *et al., Lancet* 1091-1093 (1984).
Cook, J., *et al., Ann. Clin. Biochem.* 12:219 (1975).
Daniloff, Y., *et al., Connect. Tissue. Res.* 27:187 (1992).
Eyre, D.R., *et al., Anal. Biochem.* 137:380-388 (1984).
Eyre, D.R., *et al., FEBS* 2:337-341 (1987).
Fujimoto, D., *et al., J. Biochem.* 83:863-867 (1978).
Fujimoto, D., *et al., J. Biochem.* 94:1133-1136 (1983).
Gosling, J., *Clin. Chem.* 36(8):1408 (1990).
Gunja-Smith, Z., *et al., Biochem. J.* 197:759-762 (1981).
Harlow, E., et al., Antibodies: A Laboratory Manual, Cold Spring Harbor Lab (1988).
Henkel, W., *et al., Eur. J. Biochem.* 165:427-436 (1987).
Macek, J., *et al., Z. Rheumatol.* 46:237-240 (1987).
Ogawa, T., *et al., Biochem. Biophys. Res. Commune.* 107:1252-1257 (1982).
Robins, S.P., *Biochem J.* 207:617-620 (1982a).
Robins, S.P., in "Collagen in Health and Disease" (Weiss, J.B., *et al.*, eds.) pp. 160-178, Churchill Livingstone, Edinburgh (1982b).
Robins, S.P., *Biochem. J.* 215:167-173 (1983).
Robins, S.P., *et al., Ann. Rheumatic Dis. 45*:969-973 (1986).
Robins, S.P., *et al., Biochim. Biophys. Acta.* 914:233-239 (1987).
Seibel, M.J., *et al., J. Rheumatol* 16:964-970 (1989).
Wong, S.S., Chemistry of Protein Conjugation and Cross-Linking, CRC Press, Boca Raton, Florida (1991).

### 3. Background of the Invention

There are a variety of conditions in humans which are characterized by a high level of bone resorption and by an abnormal balance between bone formation and bone resorption. Among the more common of these are osteoporosis, Paget's disease, and conditions related to the progress of benign and malignant tumors of the bone and metastatic cancers which have been transferred to bone cells from, for example, prostate or breast initial tumors. Other conditions which are associated with changes in collagen metabolism include osteomalacial diseases, rickets, abnormal growth in children, renal osteodystrophy, and a drug-induced osteopenia. Irregularities in bone metabolism are often side effects of thyroid treatments and thyroid conditions *per se*, such as primary hypothyroidism and thyrotoxicosis as well as Cushing's disease.

It has been recognized that disorders of bone resorption or other conditions characterized by an abnormal balance between bone formation and bone resorption can be detected by altered levels of pyridinium crosslinks in urine (Robins, 1982b; Macek; Black). The crosslinks, which originate from a number of collagen-containing tissues, take the form of compounds containing a central 3-hydroxy pyridinium ring in which the ring nitrogen is derived from the epsilon amino group of either lysine or hydroxylysine (Fujimoto, 1978; Robins, 1982a; Gunja-Smith; Ogawa; Eyre).

The pyridinium crosslink compounds found in urine can be grouped into four general classes: (1) free, native crosslinks having a molecular weight of about 400 daltons (Fujimoto), (2) glycosylated crosslinks and crosslink peptide forms having a molecular weight of between about 550 and 1,000 daltons (Robins, 1983), (3) crosslink peptide forms having a molecular weight between 1,000 and 3,500 daltons (Robins, 1983, 1987; Henkel; Eyre), and (4) crosslink peptide forms having a molecular weight greater than 3,500 daltons. In normal adults, these forms account for about 38% (1), 40% (2), 15% (3), and 7% (4) of total urinary crosslinks (Daniloff). About 80% of the free crosslinks (group 1 above) in normal adult urine is pyridinoline (Pyd), the ring nitrogen of which derives from hydroxylysine, and about 20% is deoxypyridinoline (Dpd), the ring nitrogen of which derives from lysine. This Pyd/Dpd ratio applies roughly to the other three classes of crosslinks in urine. The higher molecular weight crosslinks can be converted to free crosslinks by acid hydrolysis (Fujimoto, 1978).

Methods for measuring pyridinium crosslinks in urine have been proposed. One of these methods involves the measurement of total hydrolysed Pyd, i.e., Pyd produced by extensive hydrolysis of urinary crosslinks, by quantitating the hydrolysed Pyd peak separated by HPLC (Fujimoto, 1983). The relationship between total hydrolysed Pyd to age was determined by these workers as a ratio to total hydrolysed Pyd/creatinine, where creatinine level is used to normalize crosslink levels to urine concentration and skeletal mass. It was found that this ratio is high in the urine of children, and relatively constant throughout adulthood, increasing slightly in old age. The authors speculate that this may correspond to the loss of bone mass observed in old age.

Studies on the elevated levels of total crosslinks in hydrolyzed urine of patients with rheumatoid arthritis has been suggested as a method to diagnose this disease (Black, 1989). The levels of total hydrolyzed crosslinks for patients with rheumatoid arthritis (expressed as a ratio of total crosslinks measured by HPLC to creatinine) were elevated by a factor of 5 as compared to controls. However, only total hydrolysed Pyd, but not total hydrolysed Dpd, showed a measurable increase.

In a more extensive study using hydrolyzed urines, Seibel *et al*. showed significant increases in the excretion of Pyd and Dpd crosslinks relative to controls in both rheumatoid and osteoarthritis. The most marked increases for Pyd crosslinks were in patients with rheumatoid arthritis (Seibel, et al.).

Assay methods, such as those just noted, which involve HPLC quantitation of crosslinks from hydrolysed samples, or crosslink subfractions from non-hydrolysed samples, are relatively time-consuming and expensive to carry out, and may not be practical for widespread screening or monitoring therapy in bone metabolism disorders.

Immunoassays have also been proposed for measuring urinary crosslinks. U.S. Patent No. 4,973,666 discloses an assay for measuring bone resorption by detection in urine of specific pyridinium crosslinks, characterized by specific peptide extensions, associated with bone collagen. Two specific entities having peptide extensions presumed to be associated with bone collagen are described. These are obtained from the urine of patients suffering from Paget's disease, a disease known to involve high rates of bone formation and destruction. The assay relies on immunospecific binding of crosslink compounds containing the specific peptide fragment or extension with an antibody prepared against the crosslink peptide. It is not clear whether and how the concentration of crosslink peptide being assayed relates to total urinary crosslinks.

Robins has described a technique for measuring pyridinoline in urine by use of an antibody specific to hydrolysed Pyd (Robins, et al., 1986). The method has the limitation that the antibody was found to be specific for the hydrolyzed form of Pyd, requiring that the urine sample being tested first be treated under hydrolytic conditions. The hydrolytic treatment increases the time and expense of the assay, and precludes measurements of other native pyridinium crosslinks.

The pyridinium crosslink content of collagen-containing tissues is known to vary in amount and composition according to tissue type. Pyd crosslinks are found in cartilage, bone, intervertebral discs, ligaments, and the aorta. Dpd crosslinks, which are generally less prevalent than Pyd crosslinks, are found in bone, dentine, ligaments, and the aorta. The proportion of Dpd in tissue pyridinium crosslinks appears to be highest in bone, which has a Pyd:Dpd ratio of between about 3:1 and 4:1. Pyridinium crosslinks in cartilage, on the other hand, contain predominantly Pyd.

Ideally, an assay method for assessing bone metabolism, based on pyridinium crosslink levels in urine, should (a) employ a non-hydrolysed urine sample, to avoid the need for acid hydrolysis of the sample, and (b) utilize an antibody reagent to detect native free pyridinium species, for reducing the time and expense of analysis over conventional HPLC-based tests.

WO 89/04491 discloses an assay for assessing bone resorption based on measuring collagen peptides that contain pyridinium crosslinks.

WO 89/12824 teaches detection, in urine, of pyridinium crosslinks attached to original amino acids or sugars.

WO 91/10141 describes methods of diagnosing disorders of bone or connective tissue metabolism, comprising the step of determining the level of native peptide-free Pyd or Dpd crosslinks in a biological fluid of a subject, and comparing those levels to the levels in a normal subject. Preferably, the step of determining is carried out by means of an antibody or fragment thereof that is specifically immunoreactive with the native peptide-free crosslinks. However, WO 91/10141 states that antibodies raised against H-Pyd as described by Robins et al. (1982a, 1986) were not significantly cross-reactive with native peptide-free Pyd and Dpd.

The present invention provides an antibody produced by immunizing an animal with hydrolyzed pyridinoline or hydrolyzed deoxypyridinoline which may be conjugated to a carrier, said antibody being characterized by:
immunospecific binding to pyridinium crosslinks selected from native free pyridinoline and/or native free deoxypyridinoline, and
a ratio of reactivity toward said selected native free pyridinium crosslink(s) and urinary pyridinium peptides larger than 1,000 daltons in molecular weight of greater than 3:1.

In one embodiment, the antibody is specific for native free pyridinoline, and has a ratio of reactivity toward native free pyridinoline and native free deoxypyridinoline of greater than 5:1. In a second embodiment, the antibody is specific for native free deoxypyridinoline, and has a ratio of reactivity toward native free deoxypyridinoline and native free pyridinoline of greater than 25:1. In another embodiment, the antibody is specific for both native free pyridinoline and native free deoxypyridinoline. Preferably, the antibody has a ratio of reactivity toward native free pyridinoline and native free deoxypyridinoline of between 2:1 and 1:2.

More generally, the ratio of reactivity toward native free pyridinoline and native free deoxypyridinoline can be from greater than 5:1 to less than 1:25, including all ratios in between.

Preferably, the antibody is a monoclonal antibody. In other embodiments, the antibody is a polyclonal antibody which is capable of reacting immunospecifically with pyridinium crosslinks selected from the group consisting of native free pyridinoline and native free deoxypyridinoline. The polyclonal antibody has a ratio of reactivity toward the selected pyridinium crosslinks and urinary pyridinium peptides larger than 1,000 daltons in molecular weight, of greater than 3:1, preferably greater than 5:1.

The present invention also provides a diagnostic kit for use in assaying a bone collagen degradation level in a human subject, comprising:
an antibody according to the present invention; and
detection means for detecting the amount of immunocomplex formed by reaction of said antibody with such free pyridinium crosslinks.

Preferably, the detection means includes a reporter enzyme which is effective to produce a colorimetric signal, although other formats can be used. In one general embodiment, the kit includes a solid-phase support having a surface-attached binding agent which may be an antibody reagent such as described above, or native free pyridinium crosslinks. In another general embodiment, the kit employs an EMIT configuration.

The present invention further provides a method of assaying bone collagen degradation level in a human subject, comprising:
reacting a non-hydrolyzed human urine sample with an antibody in accordance with the present invention;
by said reacting forming an immunocomplex between the antibody and such selected pyridinium crosslink(s) present in the sample; and
from the amount of immunocomplex formed, determining the level of the selected pyridinium crosslink(s) in the sample, whereby a determined level which is above that characteristic of normal subjects indicates the presence of an elevated rate of bone collagen degradation level in the subject.

The urine sample may be passed through a nitrocellulose filter or other suitable separation medium to remove sample components (e.g. proteins) which may interfere with the assay.

In a preferred assay, the filtered urine sample is reacted with a binding reagent effective to form an antigen-antibody complex with the analyte, under conditions effective to bind the antibody to the solid support in proportion to the amount of analyte in the sample. Preferred filter membrane include nitrocellulose, Immobilon®-CD and Immobilon®-PSQ.

Monoclonal antibodies in accordance with the present invention can be prepared by a method that includes forming a hybridoma composed of the fusion product of (a) spleen cells from an animal immunized with hydrolyzed free pyridinoline or hydrolyzed free deoxypyridinoline attached to a carrier protein, and (b) an immortalizing fusion partner, and selecting hybridomas which are immunoreactive with the selected native free pyridinium crosslinks.

These and other objects and features of the invention will become more fully apparent when the following detailed description of the invention is read in conjunction with the accompanying drawings, in which:-
Figs. 1A-1C illustrate steps in practicing an embodiment of an assay in accordance with the invention.
Fig. 2 shows a linear regression plot of native free pyridinoline concentration measured using a monoclonal antibody reagent of the invention (y-axis), versus total (hydrolyzed) pyridinoline concentration measured by HPLC, in 44 urine samples.
Fig. 3 shows native free pyridinoline levels in normal patients compared with those in metastatic cancer patients, as measured using an assay method in accordance with the invention.
Fig. 4 shows native free pyridinoline levels in patients diagnosed with severe osteoporosis and hip fracture (group 1), osteoporosis in the absence of hip fracture (group 2), and in age-matched control patients, as measured using an assay method in accordance with the invention.
Fig. 5 shows native free deoxypyridinoline levels in normal patients (group 1), patients with metabolic bone disease conditions (group 2), and oncology patients (group 3), as measured using an assay method in accordance with the invention.
Fig. 6 shows native free deoxypyridinoline levels in osteoporosis patients before and after 1 year of estrogen treatment, as measured using an assay method in accordance with the invention.
Figs. 7A-7B illustrate steps in practicing another embodiment of an assay in accordance with the invention.
Fig. 8 shows residual enzyme activity of glucose-6-phosphate dehydrogenase (G6PDH) as a function of the number of pyridinoline or deoxypyridinoline molecules coupled to the enzyme via the coupling reagent EDC.
Figs. 9A and 9B show plots of residual enzyme activity of Pyd-labeled G6PDH in the presence of increasing concentrations of a Pyd-specific monoclonal antibody (9A) and a Pyd-specific polyclonal antibody (9B).
Fig. 10 shows a standard curve obtained using the assay configuration shown in Figs. 7A-7C.

### Detailed Description of the Invention

### I. Definitions

As used herein, the terms below have the following definitions:
"Pyd" or "pyridinoline" or "free pyridinoline" refers to the compound shown at I below, where the ring nitrogen is derived from the ε amino group of a hydroxylysyl residue, and "Dpd" or
"deoxypyridinoline" or "free deoxypyridinoline" refers to the compound shown at II below, where the ring nitrogen is derived from the ε amino group of a lysyl residue. 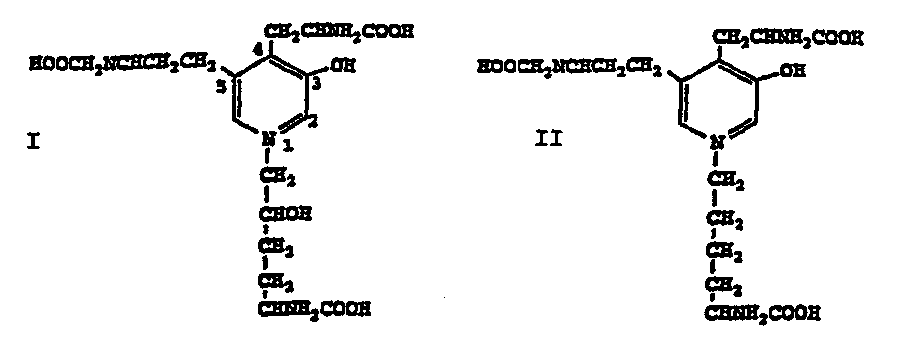
"Free crosslinks" refers to compound I or II, or to a mixture thereof.
"Glycosylated pyridinoline" or "glyco-Pyd" refers to glycosylated forms of compound I, wherein glycosyl groups are covalently bound to the aliphatic hydroxyl group of Pyd. Two glyco-Pyd crosslinks which have been identified are Gal-Pyd and Glc-Gal-Pyd, which contain the acetals shown at III and IV below, respectively. 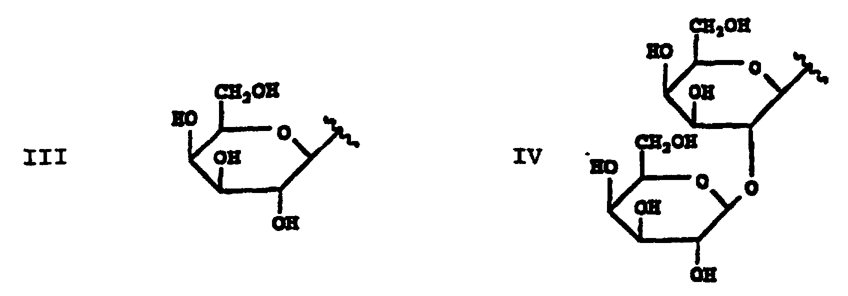
"Pyd-peptides" or "pyridinoline-peptides" refers to peptide-derivatized forms of compound I, in which one or more of the three amino acid residues in the compound is linked via a peptide linkage to additional amino acid residues. Similarly, "Dpd-peptides" or "deoxypyridinoline-peptides" refers to peptide-derivatized forms of compound II, in which one or more of the three amino acid residues in the compound is linked via a peptide linkage to additional amino acid residues.
"Pyridinium-peptides" refers to a mixture of Pyd-peptides and Dpd-peptides.
"Pyd-peptides having a molecular weight greater than 1000 daltons" or "Pyd-peptides (MW>1000)" refers to Pyd-peptides retained by a dialysis membrane having a 1,000 molecular weight cutoff.
"Dpd-peptides having a molecular weight greater than 1000 daltons" or "Dpd-peptides (MW>1000)" refers to Dpd-peptides retained by a dialysis membrane having a 1,000 molecular weight cutoff.
"Pyd crosslinks" refers to the pyridinium crosslinks in urine which contain compound I either in free or derivatized form. Pyd crosslinks include Pyd, glyco-Pyd and Pyd-peptides. Similarly, "Dpd crosslinks" refers to the pyridinium crosslinks in urine which contain compound II either in free or derivatized form. "Dpd crosslinks" include Dpd and Dpd-peptides.
"Pyridinium crosslinks" refers to pyridinium crosslinks which contain compounds I and/or II in free and/or derivatized form.
"Total H-Pyd" refers to total hydrolysed Pyd produced by hydrolyzing Pyd crosslinks to Pyd. Similarly, "total H-Dpd" refers to total hydrolysed Dpd produced by hydrolyzing Dpd crosslinks to Dpd.
"Hydrolysed-Pyd" or "H-Pyd" refers to Pyd produced by hydrolysing Pyd crosslinks in 6N HCl at 110°C for 16 hours. Similarly, "Hydrolysed-Dpd" or "H-Dpd" refers to Dpd produced by hydrolysing Dpd crosslinks in 6N HCl at 110°C for 16 hours.
"Native Pyd" or "N-Pyd" refers to Pyd obtained from urine which has not been subjected to hydrolytic conditions. Similarly, "native Dpd" or "N-Dpd" refers to free Dpd obtained from urine which has not been subjected to hydrolytic conditions.

A monoclonal antibody (or antibody reagent) which is "capable of reacting immunospecifically with pyridinium crosslinks selected from the group consisting of native free pyridinoline and native free deoxypyridinoline" refers to a monoclonal antibody which is immunospecific for native free pyridinoline, native free deoxypyridinoline, or both, relative to other antigenic materials which may be present in urine samples. The ratio of reactivity (affinity) of the monoclonal antibody toward native free pyridinoline and native free deoxypyridinoline can range from about 5:1 or greater, to about 1:25 or less, including all ratios in between.

The meaning of "ratio of reactivity toward the selected pyridinium crosslinks and urinary pyridinium peptides larger than 1,000 daltons in molecular weight, of greater than 3:1" depends on the relative specificities of the antibody for native free pyridinoline versus native free deoxypyridinoline. Where the antibody has a ratio of reactivity toward native free pyridinoline and native free deoxypyridinoline which is greater than 2:1, the quoted phrase refers to the ratio of reactivity toward N-Pyd and Pyd-peptides (MW>1000). Where the antibody has a ratio of reactivity toward native free pyridinoline and native free deoxypyridinoline which is less than 1:2, the quoted phrase refers to the ratio of reactivity toward N-Dpd and Dpd-peptides (MW>1000). For a monoclonal antibody having a ratio of reactivity toward native free pyridinoline and native free deoxypyridinoline which is between or equal to 2:1 and 1:2, the quoted phrase means that the ratio of reactivity toward N-Pyd and Pyd-peptides (MW>1000) and also the ratio of reactivity toward N-Dpd and Dpd-peptides (MW>1000) are both greater than 3:1.

### II. Preparation of Antibody Reagent

This section describes the production of monoclonal and polyclonal antibodies ("antibody reagent") which are specific for native free pyridinium crosslinks (either N-Pyd, N-Dpd, or both), as evidenced by a ratio of reactivity toward the native free pyridinium crosslink and urinary pyridinium peptides larger than 1,000 daltons in molecular weight, of greater than 3:1, and preferably greater than 5:1.

Where the antibody is for binding native free pyridinoline, the antibody preferably has a ratio of reactivity toward native free pyridinoline and native free deoxypyridinoline of greater than 5:1, preferably greater than 20:1, and more preferably greater than 100:1.

Where the antibody is for binding native free deoxypyridinoline, the antibody preferably has a ratio of reactivity toward native free deoxypyridinoline and native free pyridinoline of greater than 5:1, preferably greater than 25:1, and more preferably greater than 100:1.

Where the antibody is for binding both native free pyridinoline and native free deoxypyridinoline, the antibody preferably has a ratio of reactivity toward native free pyridinoline and native free deoxypyridinoline of between 2:1 and 1:2.

The antibody reagent of the invention preferably has a binding affinity constant for the selected pyridinium species (N-Pyd or N-Dpd) of greater than 5 × 10⁷/molar.

### A. Immunogen

The immunogen used in producing the antibody reagent is H-Dpd or H-Pyd conjugated to a carrier molecule, typically a carrier protein such as keyhole limpet hemocyanin (KLH).

Hydrolyzed Pyd or Dpd can be produced by acid hydrolysis of pyridinium crosslinks in bone collagen or urine, purified as described in Black et al., 1988, for example.

Coupling of H-Pyd or H-Dpd to a carrier protein is by standard coupling methods, typically using a bifunctional coupling agent which forms, at one coupling end, an amide linkage to one of the free carboxyl groups of H-Pyd or H-Dpd, and at the other coupling end an amide or ester or disulfide linkage to the carrier protein, according to standard methods.

Alternatively, in a preferred embodiment, the H-Pyd or H-Dpd can be directly coupled to the protein, e.g., in the presence of a water-soluble carboxyl activating agent such as EDC (1-(3-dimethylaminopropyl)-3-ethylcarbodiimide), also according to well known methods. The latter approach is illustrated in Example 3, which describes the coupling of H-Dpd to keyhole limpet hemocyanin (KLH) by EDC activation. General coupling reactions for derivatizing a carrier protein with a peptide antigen are given in Harlow (1988), pp. 77-87, and in Wong (1991).

### B. Monoclonal Antibody Reagent

To prepare a monoclonal antibody reagent, the immunogen described above is used to immunize an animal, such as a mouse, from which antigen-specific lymphocytes can be obtained for immortalization. One animal that has been found suitable is the "autoimmune" MRL/MpJ-lpr mouse available from Jackson Laboratory (Bar Harbor, ME).

Where an antibody which is specific for N-Pyd is desired, an H-Pyd-immunogen is typically used. Likewise, where an antibody which is specific for N-Dpd is desired, an H-Dpd-immunogen is typically used. An antibody which recognizes both Pyd and Dpd may be obtained using an H-Pyd-immunogen or an H-Dpd-immunogen.

B1. N-Pyd Monoclonal Antibody. For producing a monoclonal antibody reagent which is specific for N-Pyd, mice can be immunized using a series of injections of H-Pyd-KLH immunogen, as outlined in Example 4. About 8 weeks after initial immunization, spleen cells are harvested and fused with a P3X63Ag8.653 myeloma cell line. Selection for successful fusion products can be performed in HAT in conditioned S-DMEM medium, according to published methods (see, generally, Harlow, pp. 196-212). Successful fusion products are then screened for immunoreactivity with N-Pyd, using a competitive immunoassay format similar to that described in Example 8. Cell lines which show high affinity binding to N-Pyd are subcloned by limiting dilution and further screened for production of antibodies with high binding affinity for N-Pyd. One subcloned cell line obtained by the procedure above and which gave high antibody affinity for N-Pyd is designated herein as Mab-XXV-3G6-3B11-1A10. Samples of this cell line have been deposited with the American Type Culture Collection, 12301 Parklawn Dr., Rockville MD 20852, and have been assigned ATCC No. HB11089.

To produce the antibody reagent, the hybridoma cell line is grown in a suitable medium (Harlow, pp. 247-270), such as Dulbecco's modified Eagle's medium (DMEM) supplemented as described in the Materials and Methods section below. Monoclonal antibodies ("Mabs") are harvested from the medium and can be concentrated and stored according to published methods (Harlow pp. 271-318).

As noted above, an important feature of the present invention is the specificity of the antibody reagent for N-Dpd and N-Pyd relative to larger molecular weight pyridinium crosslinks in urine. The relative specificity of the antibody reagent for N-Pyd, N-Dpd, and other urinary pyridinium crosslinks can be determined by a competitive binding assay for N-Pyd, as detailed in Example 10.

Briefly, various purified crosslink samples, including N-Pyd and N-Dpd, as well as an amino acid mixture containing the 20 common amino acids in equimolar amounts (150 iM each), are reacted with a limiting amount of the antibody reagent over a solid-phase support having attached N-Pyd under conditions in which the pyridinium crosslinks in the sample compete with the support-bound N-Pyd for binding to the antibody. The extent of binding of antibody to the solid-support provides a measure of the relative reactivities of the sample crosslinks for the antibody reagent.

In accordance with the procedure outlined in Example 10, the levels of binding of N-Pyd, N-Dpd, Pyd-peptides (MM>1,000), and an amino acid mixture (150 µM each of the common 20 amino acids), to monoclonal antibodies from cell line Pyd XXV-3G6-3B11-1A10 were examined. The apparent Pyd concentration of each sample was determined using standard curves established using purified N-Pyd. The percent-reactivity of each sample was calculated as a ratio of apparent concentration (measured using. the N-Pyd standard curve above) to total Pyd crosslink concentration in the sample determined by HPLC for total H-Pyd (times 100), or to total Dpd-crosslink concentration as determined by HPLC for total H-Dpd (times 100) in the case of the N-Dpd sample. The results are shown in Table 1, where reactivity with N-Pyd has been defined as 100%.

**Table 1**

| **Cross-Reactivity of N-Pyd Monoclonal Antibody** | |
|---|---|
| N-Pyd | 100% |
| N-Dpd | 16% |
| Pyd-Peptide (>1000) | <1% |
| Amino Acid Mixture (150 µM) | <1% |

As seen, the monoclonal antibody reagent is highly selective for N-Pyd relative to N-Dpd, showing a ratio of reactivity toward native free pyridinoline and native free deoxypyridinoline that is greater than 3:1, and in the present case, greater than 5:1. The reagent is also selective for N-Pyd over the pyridinium-peptide forms tested (quantitated for total Pyd content), showing a ratio of reactivity toward pyridinoline peptides larger than 1,000 daltons in molecular weight, of greater than 100:1. In addition, the reagent shows minimal cross reactivity (<1%) with the amino acid mixture tested.

More generally, the Mab reagent which is specific for N-Pyd has a reactivity toward native free pyridinoline (N-Pyd) and Pyd-peptides (MW>1000), of greater than 5:1, preferably greater than 10:1, more preferably greater than 25:1, and in the present case, greater than 100:1, as measured by the above assay.

B.2 N-Dpd Monoclonal Antibody. For producing a monoclonal antibody reagent which is specific for N-Dpd, the above procedure for obtaining N-Pyd Mabs can be used, except that H-Dpd-KLH is used as immunogen, and immunoreactivity screening is done with an assay for N-Dpd. One subcloned cell line obtained by this procedure, and which gave high antibody affinity for N-Dpd, is designated herein as Mab-Dpd-II-7B6-1F4-1H11 (see Example 5).

The antibody reagent is prepared from the hybridoma cell line and stored by the same general procedures described above for N-Pyd Mabs.

The relative specificity of the antibody reagent for N-Dpd, N-Pyd, and other urinary pyridinium crosslinks can be determined by the approach described above (section B.1), but using a solid-phase support having attached N-Dpd.

In accordance with the procedure outlined in Example 10, the levels of binding of N-Dpd, N-Pyd, Dpd-peptides (MW>1,000), and an amino acid mixture (150 µM each of the common 20 amino acids), to monoclonal antibodies from cell line Mab-Dpd-II-7B6-lF4-1H11 were examined. The apparent Dpd concentration of each sample was determined using standard curves established using purified N-Dpd. The percent reactivity of each sample was calculated as a ratio of apparent N-Dpd concentration (measured using the N-Dpd standard curve above) to total Dpd-crosslink concentration in the sample determined by HPLC for total H-Dpd (times 100), or to total Pyd-crosslink concentration as determined by HPLC for total H-Pyd (times 100) in the case of the N-Pyd sample. The results are shown in Table 2, where reactivity with N-Dpd has been defined as 100%.

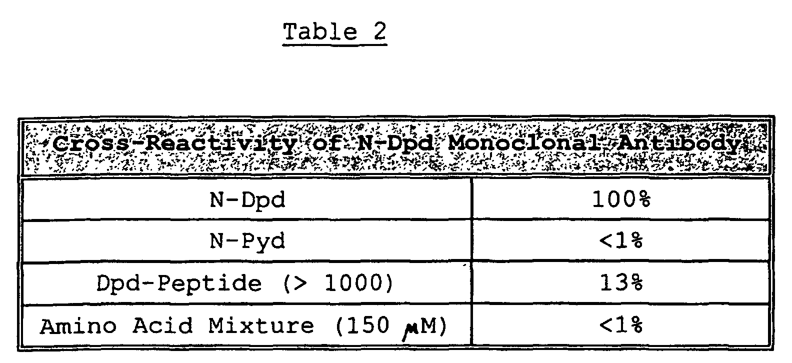

As seen, the monoclonal antibody reagent is highly selective for N-Dpd relative to N-Pyd, showing a ratio of reactivity toward native free deoxypyridinoline and native free pyridinoline that is greater than 100:1. The reagent is also selective for N-Dpd over the pyridinium-peptide forms tested (quantitated for Dpd content), showing a ratio of reactivity toward deoxypyridinoline peptides larger than 1,000 daltons in molecular weight, that is greater than 3:1, and preferably, greater than 5:1. In addition, the reagent shows minimal cross reactivity (<1%) with the amino acid mixture tested.

More generally, the Mab reagent which is specific for N-Dpd has a reactivity toward native deoxypyridinoline (N-Dpd) and Dpd-peptides (MW>1000), of greater than 5:1, preferably greater than 10:1, more preferably greater than 25:1, and in the present case, greater than 100:1, as measured by the above assay.

B.3 Monoclonal Antibodies Which Bind N-Pyd and N-Dpd With Comparable Affinities. For producing a monoclonal antibody reagent which binds N-Pyd and N-Dpd with comparable affinity, the procedures described above for obtaining N-Pyd Mabs and N-Dpd Mabs can be used. The immunogen may be Pyd-KLH or Dpd-KLH, and immunoreactivity screening is done with separate assays for N-Pyd and N-Dpd. One subcloned cell line obtained by the procedure above, using H-Dpd-KLH as immunogen, and which gave high antibody affinity for both N-Dpd and N-Pyd, is designated herein as Mab Pyd/Dpd-V-6H2-2H4-1E4 (see Example 6).

The antibody reagent is prepared from the hybridoma cell line and stored by the same general procedures described above for the N-Pyd Mabs.

The relative specificity of the antibody reagent for N-Dpd, N-Pyd, and other urinary pyridinium crosslinks can be determined by the procedure described above (sections B.1 and B.2). In the present case, for antibodies produced using the Pyd/Dpd-V-6H2-2H4-1E4 cell line, the percent reactivity of each sample was calculated as a ratio of apparent N-Dpd concentration (measured using the N-Dpd standard curve above) to total Dpd crosslink concentration in the sample determined by HPLC for total H-Dpd (times 100), or to total Pyd crosslink concentration determined by HPLC for total H-Pyd in the case of the N-Pyd sample. The results are shown in Table 3, where reactivity with N-Dpd has been defined as 100%.

**Table 3**

| **Cross-Reactivity of Pyd/Dpd Monoclonal Antibody** | |
|---|---|
| N-Dpd | 100% |
| N-Pyd | 102% |
| Dpd-Peptide (>1000) | 1% |
| Pyd-Peptide (>1000) | 11% |
| Amino Acid Mixture (150 µM) | 5% |

As seen, the monoclonal antibody reagent recognizes N-Dpd and N-Pyd with comparable affinities, with a cross-reactivity ratio close to 1:1. The reagent is also selective for N-Dpd over the pyridinium-peptide forms tested (both Pyd and Dpd peptides), showing a ratio of reactivity toward pyridinium peptides larger than 1,000 daltons in molecular weight, of greater than 3:1, and in the present case, greater than 9:1. In addition, the reagent shows minimal cross reactivity (5%) with the amino acid mixture tested.

More generally, the Pyd/Dpd-specific Mab reagent has a reactivity toward native free pyridinoline (N-Pyd) and native free deoxypyridinoline (N-Dpd) of between 2:1 and 1:2.

B.4 Affinity Chromatography. The antibody reagent of the invention can also be used in an affinity chromatography matrix, in accordance with standard affinity chromatography methods, for binding and collecting native free pyridinium crosslinks derived from collagen tissues, e.g., by passing pooled urine through such a matrix. For isolation of N-Pyd, an antibody reagent which is specific for N-Pyd is used in the matrix. Alternatively, for isolation of both N-Pyd and N-Dpd, an antibody which binds both with comparable affinities is employed. The native free crosslinks obtained can be further purified by methods described in Examples 1 and 2, as necessary.

### C. Polyclonal Antibodies

Polyclonal antibody preparation is by conventional techniques, including injection of the immunogen into suitable mammalian subjects, such as rabbits or mice, according to immunological protocols generally known in the art, e.g., Harlow, pp. 93-115. Typically, rabbits are injected subcutaneously with the immunogen in an adjuvant, and booster immunizations are given by subcutaneous or intramuscular injection every 2-3 weeks; mice may be injected intraperitoneally according to a similar schedule. Blood is collected at intervals, e.g. 1-2 weeks after each immunization injection. Antisera may be titrated to determine antibody formation with respect to N-Pyd or N-Dpd, according to standard immunoprecipitation methods (Harlow, pp. 423-470). Details of one method for producing polyclonal antibodies in rabbits are given in Example 19.

The binding affinity constant for polyclonal antisera can be determined by known methods (e.g., by Scatchard analysis using an immunoprecipitation or ELISA assay), and represents an average binding affinity constant value for the antibodies in the antisera which are specific against the selected pyridinium species. Polyclonal antibodies obtained from rabbit VI-8 have a binding constant for N-Pyd of about 1 × 10⁸, as determined by Scatchard analysis.

The relative binding specificity of the antibody reagent for the selected pyridinium species and for other pyridinium crosslinks can be determined by a competitive binding assay such as described section B above and detailed in Example 10. Table 3.5 shows the relative binding specificities of anti-Pyd antiserum obtained from rabbit VI-8, where reactivity with N-Pyd has been defined as 100%.

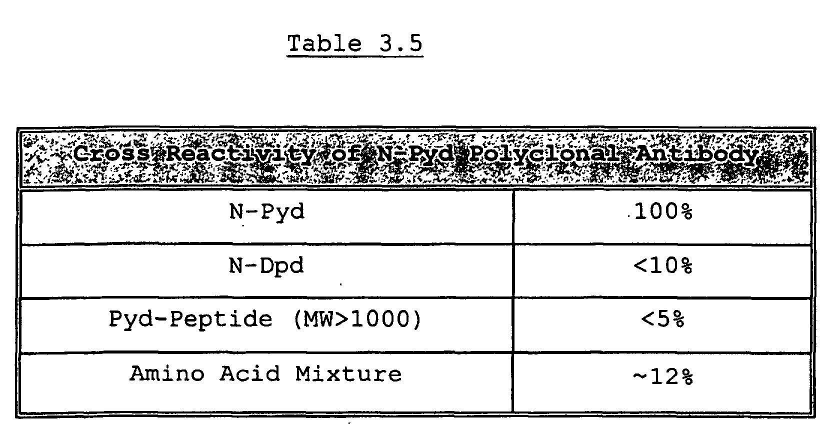

As seen, the antibody reagent is specific for N-Pyd, showing less than 10% cross-reactivity with N-Dpd, less than 5% cross-reactivity with Pyd-peptides (MW>1000), and moderate (∼12%) cross-reactivity with the amino acid mixture. In accordance with one aspect of the invention, the polyclonal antibody reagent has a reactivity toward a selected native free pyridinium species (N-Pyd, N-Dpd, or both) and urinary pyridinium peptides larger than 1,000 daltons in molecular weight, of greater than 3:1, and preferably greater than 5:1, as measured by the above antigen-competition assay.

### III. Immunoassay Kit

In another aspect, the invention includes a diagnostic kit for use in assaying collagen degradation levels in a human subject. The kit includes (a) an antibody of the invention, as described in section II above and (b) detection means for detecting the amount of immunocomplex formed by reaction of the antibody reagent with the free pyridinium crosslink(s). In a preferred embodiment, the detection means is effective to produce a colorimetric signal, i.e., a signal that can be measured spectrophotometrically.

In one general embodiment, the kit includes a solid-phase support having surface-attached binding molecules effective to bind a reporter group in the detection reagent in proportion to the amount of native free pyridinium crosslinks in the sample which are immunoreactive with the antibody reagent.

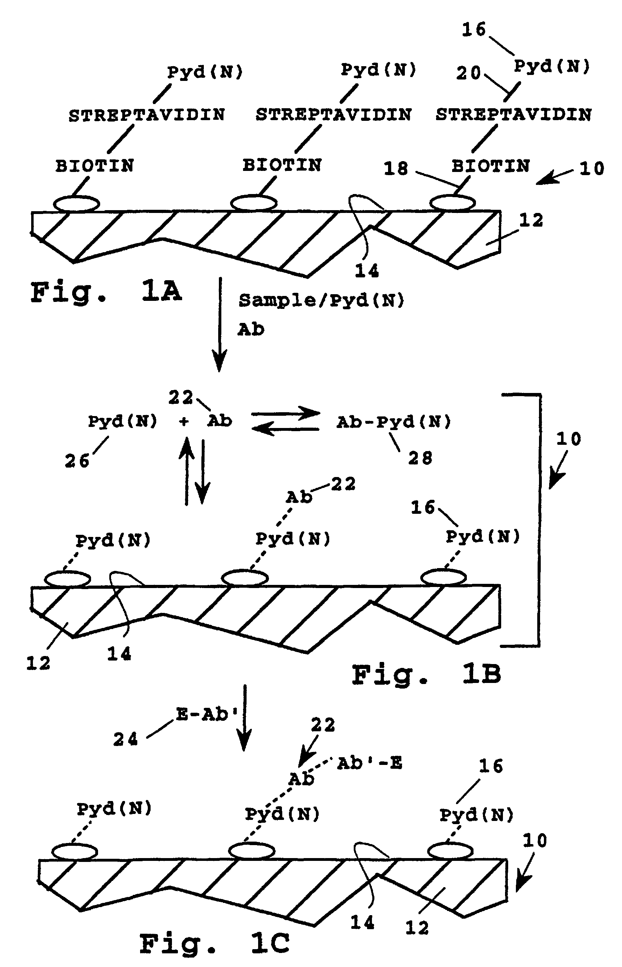

For the purpose of illustration, a specific embodiment of such a kit, for measuring N-Pyd in a sample, is shown at 10 in Figs. 1A-1C. A solid-phase support 12 in the kit has a surface to which the binding agent can be adsorbed or chemically attached. A variety of glass and polymer resin supports having chemically derivatizable groups, or surfaces effective in protein adsorption are available. In one preferred embodiment, the kit provides 96 assay wells in a microtitre plate, where the well surfaces form the solid-phase support surfaces in the kit.

The binding agent in kit 10 is N-Pyd, indicated by Pyd(N) molecules in the figures, such as at 16. The binding agent is attached to the solid phase, in this case, each of the wells in a 96-well microtitre plate, by first adsorbing an ovalbumin-biotin complex, such as complex 18 in Fig. 1A, to the well surfaces, then attaching an N-Pyd-streptavidin complex, such as complex 20, to the adsorbed biotin. Alternatively, adsorption of an ovalbumin-biotin complex can be omitted, and N-Pyd-streptavidin can be bound directly to the solid support. Methods for forming an N-Pyd coated microtitre plate are detailed in Examples 8 and 9.

The antibody reagent in the kit is indicated at 22 in Figs. 1B and 1C, and includes the monoclonal reagent described in the section above. As shown in Fig. 1B, pyridinium crosslinks in a sample, such as the N-Pyd crosslink indicated at 26, competes with surface-bound N-Pyd for binding to the antibody reagent. The immunocomplex formed by reaction of the antibody reagent with sample crosslinks is indicated at 28 in this figure.

The detection reagent in the kit is a reporter-labeled second antibody, indicated at 24 in Fig. 1C, which is effective to bind to antibody reagent which is itself bound to N-Pyd attached to the solid support. Reporter-labeled antibodies, such as enzyme-labeled antibodies, are commercially available or readily constructed (Harlow, pp. 319-358) for a variety of reporter moieties. One preferred enzyme in an enzyme-labeled antibody is alkaline phosphatase, which can react with a p-nitrophenylphosphate substrate to produce a colored product having a strong absorption peak at 405 nm.

The reporter-labeled second antibody is typically an anti-IgG antibody, such as an anti-mouse IgG antibody, where the monoclonal antibody reagent in the kit is obtained from immunized mice. Here, the antibody reagent (which is immunoreactive with N-Pyd as above) is "reporter-labelable", since the antibody reagent can become labeled by reaction with the reporter-labeled second antibody. Other instances of a reporter-labelable antibody reagent include a biotin- or streptavidin-labeled antibody which can be reacted with a reporter-labeled streptavidin or biotin-labeled partner for detection purposes.

In an alternative embodiment, the detection reagent can be the anti-Pyd antibody reagent itself, labeled with a reporter, such as an enzyme.

The detection means in the kit may also include necessary substrates or the like needed for detection of the reporter.

In an alternative embodiment of a kit having a solid-phase support, the binding agent (binding molecule) attached to the support is the antibody reagent described in Section II. The antibody may be attached to the solid support by a variety of known methods, including chemical derivatization or high-affinity binding of the antibody by support-bound protein A or anti-IgG antibody, for example, according to standard methods. In this embodiment, the kit may additionally include a pyridinoline reagent which is effective to compete with N-Pyd in a sample for binding to the antibody reagent on the support. For detection purposes, the pyridinoline reagent may include a reporter-label attached covalently to pyridinoline (i.e., the reagent can be a reporter-labeled pyridinoline). Alternatively, the pyridinoline reagent may be reporter-labelable, in that the pyridinoline reagent can include Pyd conjugated to an agent such as biotin or streptavidin, for example, for recognition by a corresponding reporter-labeled streptavidin or biotin molecule.

In another specific embodiment, the kit employs an EMIT configuration (enzyme multiplied immunoassay technique, Figs. 7A-7C). In this approach, sample analyte competes with an analyte-labeled enzyme for binding to an analyte-specific antibody, under conditions where binding of the antibody to the labeled enzyme leads to a decrease in the catalytic activity of the enzyme. Thus, the observed catalytic activity of the enzyme is proportional to the level of analyte in the sample, with higher analyte levels giving rise to higher enzyme activity.

With reference to Figs. 7A-7C, the kit includes a pyridinium-labeled enzyme 32 which is formed from an enzyme 34 and one or more pyridinium (i.e., free pyridinoline or free deoxypyridinoline) labels 36. In the embodiment shown in the figures, the pyridinium label 36 is free pyridinoline.

Enzyme 34 is capable of catalyzing the formation of a detectable product which, preferably, can be measured spectrophotometrically. An exemplary enzyme which is suitable for use in the kit is glucose-6-phosphate dehydrogenase (G6PDH) from Leuconostoc mesenteroides.

Also included is a pyridinium-specific antibody 38 which is capable of reacting immunospecifically with pyridinium label 36 and with an analyte pyridinium species 40. In the embodiment shown in Figs. 7A-7C, label 36 and analyte pyridinium species 40 are free pyridinoline.

Coupling between enzyme 34 and label 36 can be via direct or indirect linkage by use of a variety of coupling agents known in the art (e.g., Wong, 1991). For example, in a preferred embodiment, reaction of enzyme 34 with pyridinium label 36 (e.g., pyridinoline) in the presence of a carbodiimide reagent leads to direct coupling of a carboxylate or amino group in label 36 with an amino or carboxylate group in enzyme 34. Reaction conditions for forming such a direct linkage are provided in Example 15. Alternatively, label 36, enzyme 34, or both can be reacted with a coupling reagent which is effective to form a bridging group of a selected length between the label and the enzyme (i.e., via indirect coupling). In the procedure described in Example 16, pyridinoline is reacted with the coupling agent, sulfo-SMCC (referred to as SMCC hereinafter), to form a maleimide-containing pyridinoline adduct. This adduct can then be reacted with amino or sulfhydryl groups on the surface of enzyme 34 to form labeled enzyme 32. As illustrated in Example 16, the enzyme may be derivatized, e.g., with N-succinimidyl S-acetylthioacetate (SATA), to introduce additional sulfhydryl groups which are reactive with the pyridinoline adduct. A similar approach using the coupling reagent SPDP is described in Example 17.

Desirable properties of labeled enzyme 32 include (1) that the labeled enzyme retains a substantial amount of catalytic activity (i.e., greater than about 5%) with respect to the activity of unlabeled enzyme, and (2) that binding of a pyridinium-specific antibody to pyridinium label 36 results in a detectable decrease in the activity of labeled enzyme 32. Accordingly, reactions for preparing labeled enzyme may be carried out in the presence of an active site ligand (e.g., a substrate or substrate analog) to minimize loss of activity due to labeling of active site residues in the enzyme. In addition, the type(s) of enzyme surface groups that are to be labeled with label 36 can be selected by suitable choice of coupling reagent and reaction conditions, as known in the art (Wong, 1991).

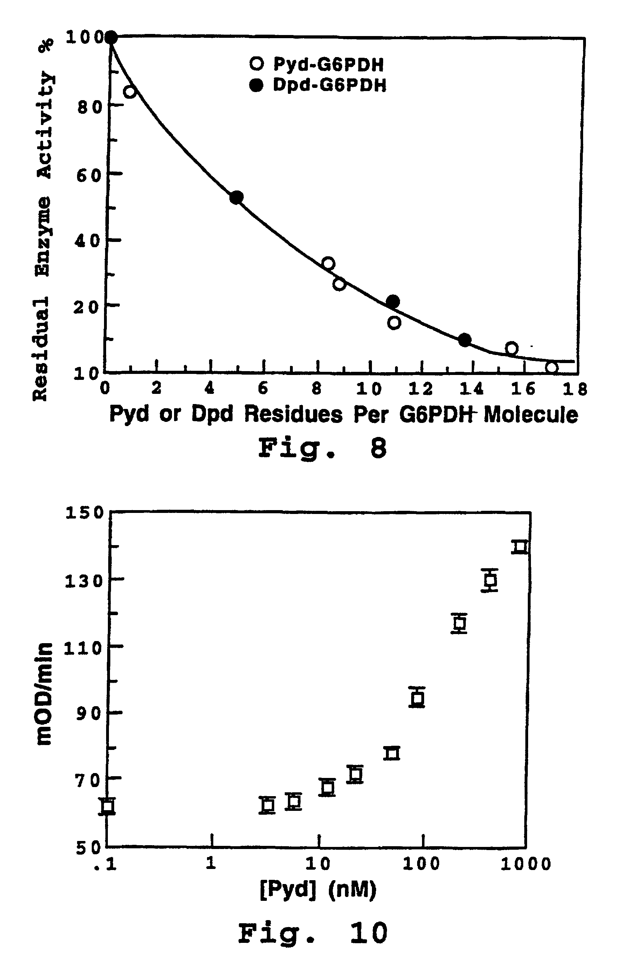

In preparing a labeled enzyme which is suitable for use in the kit, several labeled enzyme candidates should be prepared using different coupling reagents and a range of pyridinium label:enzyme ratios. Although high ratios of pyridinium label:enzyme increase the likelihood of labeling a residue near the active site (so that binding of antibody at that site will decrease catalytic activity), such high ratios can also lead to a greater loss of catalytic activity, as can be seen from Fig. 8. The optimal ratio of pyridinium label:enzyme with respect to enzyme activity is typically determined based on additional considerations discussed below.

Antibody 38 may be a polyclonal or monoclonal antibody-prepared by the general methods discussed in section II above. Typically, the specificity of the antibody with respect to native free pyridinoline, deoxypyridinoline, and higher molecular weight pyridinium peptide forms is determined as discussed in section II, and illustrated in Example 10.

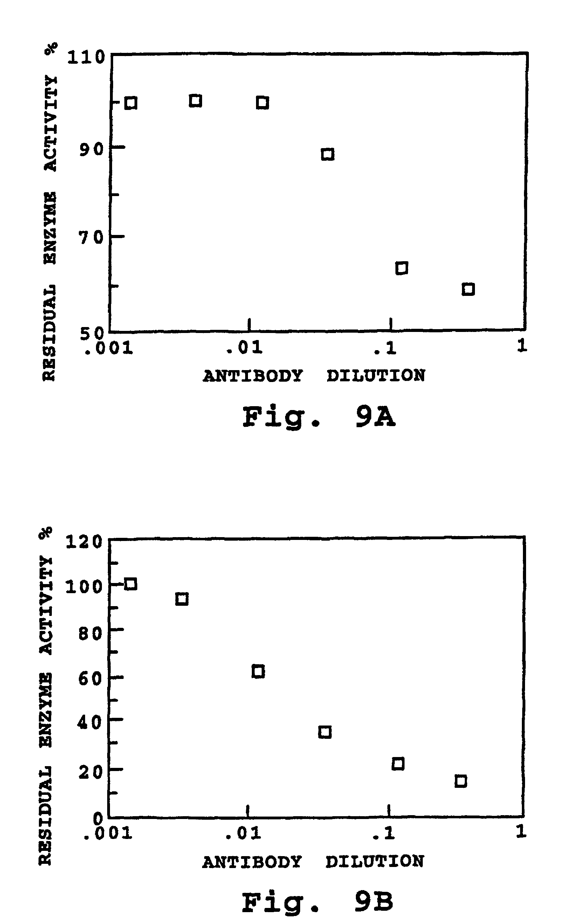

As noted above, binding of antibody to labeled enzyme 32 leads to a detectable decrease in the activity of the enzyme. Preferably, an excess of antibody 38 can reduce the catalytic activity of labeled enzyme 32 by at least 10%, and more preferably, by at least 20 to 30%. The ability of an antibody preparation to reduce catalytic activity can be assessed by the approach illustrated in Figures 9A and 9B. Fig. 9A shows the activity of Pyd-labeled G6PDH (-8 molecules of H-Pyd per molecule of G6PDH) as a function of increasing concentrations of a Pyd-specific monoclonal antibody (V-6H2) prepared as discussed in section II.B.1. As can be seen, enzyme activity dropped from 100% in the presence of a 100-fold antibody dilution to about 60% in the presence of a 10-fold antibody dilution (i.e., a decrease of 40%). With reference to Fig. 9B, which shows data for a Pyd-specific polyclonal antibody (VI-8-19), increasing the concentration of antibody from a 400-fold dilution to a 10-fold dilution led to an activity loss of about 80%.

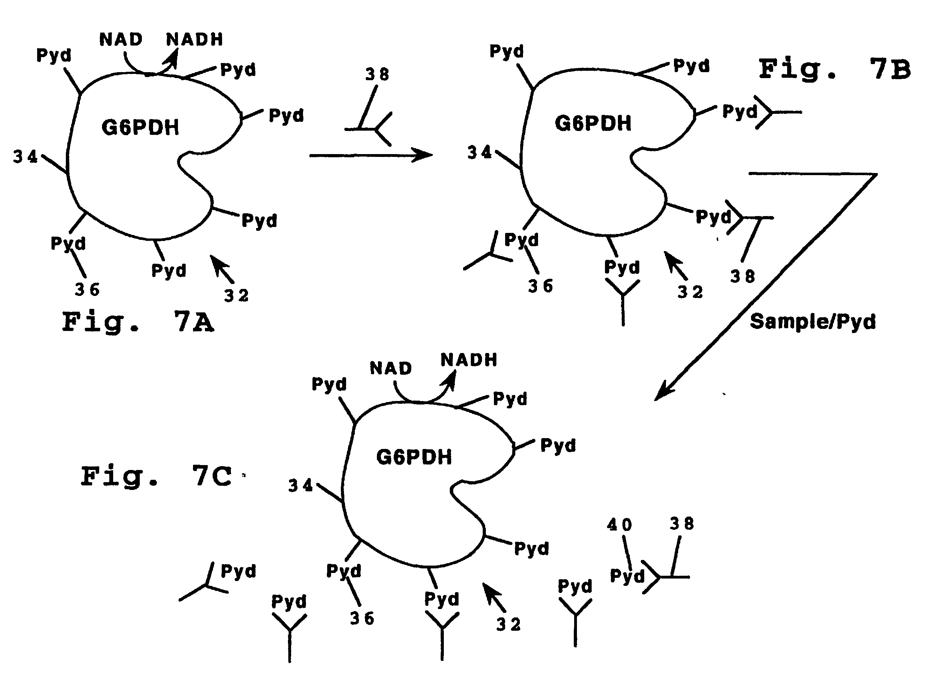

Kit 30 may also include detection reagents for measuring the catalytic activity of enzyme 32 in the presence of antibody 38 and analyte 40. In the kit illustrated in Figure 7, where enzyme 32 is G6PDH, the detection reagents include glucose 6-phosphate and the oxidized form of nicotinamide adenine dinucleotide (NAD⁺), for producing the reduced form of nicotinamide adenine dinucleotide (NADH) which is strongly absorbent at 340 nm. In a preferred embodiment, where the kit provides a multi-well microtitre plate, the catalytic activity of enzyme 32 is measured using a microplate reader capable of measuring absorbance as a function of time.

Use of an exemplary EMIT-based assay kit is detailed in Example 18. In brief, a 50 µL aliquot of pyridinoline standard or diluted urine sample is added to each well of a microtitre plate, followed by 100 µL of suitably diluted antibody. After the plate has been incubated at room temperature for a selected time (e.g., 2 minutes), 100 ul of diluted EDC-coupled pyridinoline-G6PDH conjugate (8 Pyd/G6PDH molecule) is added to each well, followed by incubation at room temperature (e.g., for 5 minutes). Following the last incubation period, 60 uL of a substrate solution containing glucose-6-phosphate and NAD⁺ is added to each well, and G6PDH activity is measured at 340 nm using a microplate reader. The activities measured with the various Pyd standards are used to construct a Pyd calibration curve (e.g., see Fig. 10) which can be used to determine the levels of Pyd in the urine samples. It can be appreciated that the order in which the sample, antibody reagent, and enzyme are added together can be different from that used in Example 18.

Although the kits above are illustrated for assay of N-Pyd, it can be appreciated that similar formats can be used where the kit is for measurement of N-Dpd, using a N-Dpd-specific antibody reagent, or for measurement of the sum of N-Pyd and N-Dpd, using an antibody reagent which binds N-Pyd and N-Dpd with comparable affinities.

In addition, it can be appreciated that the kit of the invention can be adapted to a number of other homogeneous and heterogeneous assay formats, including formats based on radiotracers, coupled enzymes, fluorescence, and chemiluminescence.

### IV. Immunoassay Method

The immunoassay method of the invention provides a method for assaying bone collagen degradation activity in a human subject. The method involves reacting a urine sample from the subject with the antibody reagent described in section II above, forming an immunocomplex between the antibody and native free pyridinoline in the sample, and measuring the amount of immunocomplex formed.

Preferably, as discussed further in Section V, the urine sample is contacted (e.g., via filtration) with a nitrocellulose filter or membrane, prior to reacting the sample with the antibody reagent, to reduce levels of substances in the sample which may interfere with the measurement of N-Pyd.

As indicated in Section III above, the reaction of the urine sample with the antibody reagent may be carried out in a solid-phase format, using a variety of configurations, or in a homogeneous assay format. For illustrative purposes, the immunoassay method is described below with reference to an assay format involving a solid-phase support, like that shown in Figs. 1A-1C.

In an exemplary embodiment of the method, a known volume, typically 10-100 µl, of the urine sample is added to the N-Pyd-coated solid support, e.g., the wells in a microtitre plate, followed by addition of a known volume, typically 50-200 µl, of antibody reagent, at a known dilution. The mixture on the solid support surface is then incubated, preferably under conditions effective to achieve equilibrium between the antibody, sample pyridinium crosslinks, and surface-bound Pyd. In the method detailed in Example 8, the incubation is overnight at 2-8°C.

After incubation, the solid support is washed several times to remove antibody not specifically bound to the support, and then incubated with a reporter-labeled anti-IgG antibody or the like, effective to bind specifically to support-bound antibody. Conveniently, for monoclonal antibodies obtained using mice, the reporter-labeled antibody is goat anti-mouse IgG conjugated to alkaline phosphatase. After a short incubation time, the support is again washed to remove non-specifically bound material, and the level of enzyme bound to the support is determined by addition of enzyme substrate, with spectrophotometric determination of converted substrate.

In a typical assay for measuring N-Pyd, N-Pyd standards containing increasing concentrations of N-Pyd are added in duplicate to some of the wells, for purposes of generating an N-Pyd concentration standard curve. Up to 40 samples are then added in duplicate to remaining wells, and the wells are then assayed as above. The standard curve is used for determining the urinary concentration of N-Pyd. The measured concentrations are preferably expressed as a ratio of N-Pyd/creatinine, to normalize the samples for variations in urine concentration and body mass. Urine creatinine concentrations can be assayed by standard methods, such as those based on reaction with alkaline picrate (Cook, 1975).

Assays for measuring N-Dpd in urine are carried out as above, but using an N-Dpd standard curve for determining the concentration of N-Dpd in the sample. Where the assay method measures the sum of N-Pyd and N-Dpd, by means of an antibody reagent that binds N-Pyd and N-Dpd with comparable affinities, it can be appreciated that a standard curve based on one of either N-Pyd or N-Dpd is sufficient in the method, once the relative affinities of the antibody reagent for N-Pyd and N-Dpd have been established.

The Pyd can be native Pyd (N-Pyd) or hydrolyzed Pyd (H-Pyd). Likewise, the Dpd can be native Dpd (N-Dpd) or hydrolyzed Dpd (H-Dpd). For obtaining N-Dpd or N-Pyd, gross separation of N-Dpd or N-Pyd from other pyridinium compounds in urine can be achieved by fractionation of urine, as described in Example 2. Briefly, a concentrate of urine is applied to a Sephadex® G-10 column, and the total pyridinium-containing fractions are eluted. The eluate is then applied to a column of phosphocellulose equilibrated with sodium citrate, and eluted with salt, yielding free crosslinks in a single peak. As the sample is not subjected to hydrolysis conditions, the peak contains not only the N-Dpd and N-Pyd forms ("free crosslinks"), but also glyco-Pyd, including Gal-Pyd and Glc-Gal-Pyd as described above. Further purification is then conveniently conducted by standard methods, for example, using ion exchange on sulfonated polystyrene beads, or HPLC. Typical protocols for this separation are found, for example, in Black, *et al.*, 1988, Seibel, *et al*., 1989, and detailed in Example 2.

Example 8 illustrates a typical procedure for assaying urine pyridinium levels in accordance with the invention, using a monoclonal antibody specific for N-Pyd, produced by the Pyd XXV-3G6-3B11-1A10 cells described in Section II.B.1 above. The assay was carried out in two 96-well microtitre plates, with 12 wells in each plate being used for duplicate samples of 6 N-Pyd standards, and 44 wells in each plate being used for 22 duplicate urine samples from post-menopausal women. A standard curve for N-Pyd generated from the six N-Pyd standards was used to calculate, for each unknown sample, the concentration (in nM) of sample pyridinium crosslinks which are immunoreactive with the assay antibody. The data from Table 1 above indicate that the measured pyridinium concentration is due almost entirely to N-Pyd in the sample, with only a minor contribution from N-Dpd.

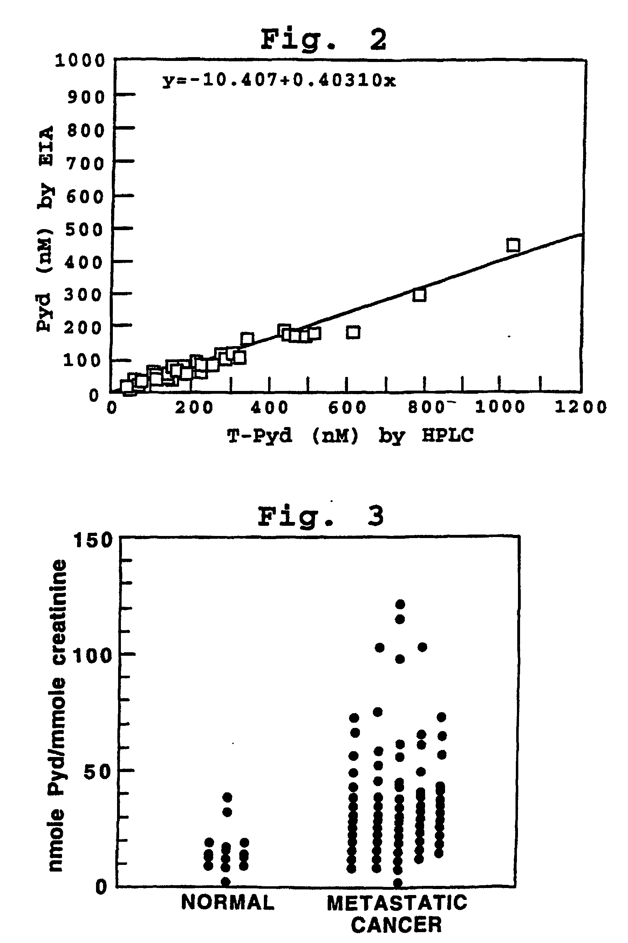

The measured pyridinium crosslink concentrations from the 44 samples were plotted against the total H-Pyd concentrations, measured by HPLC, giving the scatter plot shown in Fig. 2. The best-fit regression line in the figure is given by the equation y = -10.407 + 0.40310x. Thus, the pyridinium crosslink concentration measured in the assay is related to the concentration of total hydrolysed pyridinoline in the sample by a linear relationship with a slope of between 0.3-0.5, and specifically in this set of data, a slope of 0.403. The linear relationship between measured pyridinium crosslink concentration and total H-Pyd, and the high correlation (r=0.982) between the two, permits for accurate determination of total Pyd values in the assay from the measure pyridinium crosslink levels.

It will be appreciated how the N-Pyd levels measured in the assay can be used to provide an index for the determination of the metabolic status of tissues which generate collagen-derived crosslinks when degradation occurs. As discussed above, a variety of abnormal or pathological bone metabolic conditions are characterized by changes in both N-Pyd and total Pyd in human urine samples. Further, changes in N-Pyd provide a good measure of changes in N-Dpd as well. This latter point is illustrated by the data in Table 4 below, which shows N-Pyd and N-Dpd levels, measured by HPLC from purified N-Pyd and N-Dpd crosslinks in a variety of urine samples.

**Table 4**

| **Patient Group** | **N-Pyd** | **N-Dpd** |
|---|---|---|
| | **(nmol/mmol creatinine)** | |
| Normal controls | 10.3 ± 1.0 | 3.27 ± 0.57 |
| Osteoporosis | 19.6 ± 2.3 | 5.90 ± 0.68 |
| Paget's Disease | 62.5 ± 11.2 | 19.3 ± 3.83 |
| Hyperparathyroidism | 55.9 ± 14.2 | 16.3 ± 4.81 |
| Rheumatoid arthritis | 38.8 ± 8.36 | 8.92 ± 2.08 |
| Osteoarthritis | 25.8 ± 3.22 | 6.10 ± 0.83 |

These results show dramatically elevated levels of the free crosslinks in patients known to be suffering from diseases characterized by excessive breakdown of connective tissue.

Table 5 shows the proportions of N-Pyd and N-Dpd as a percentage of the respective total crosslink measured after hydrolysis in the different patient groups.

**Table 5**

| **Patient Group** | **% N-Pyd** | **% N-Dpd** |
|---|---|---|
| Normal controls | 43.8 ± 2.5 | 50.1 ± 5.4 |
| Osteoporosis | 41.7 ± 2.0 | 42.7 ± 2.6 |
| Paget's Disease | 46.5 ± 2.4 | 47.4 ± 4.1 |
| Hyperparathyroidism | 48.7 ± 6.8 | 46.2 ± 6.9 |
| Rheumatoid arthritis | 38.1 ± 2.6 | 43.3 ± 1.8 |
| osteoarthritis | 43.4 ± 3.9 | 47.0 ± 2.2 |

Since, as shown in Table 5, the percentage of N-Pyd (as a percentage of total Pyd) and N-Dpd (as a percentage of total Dpd) is relatively unchanged in patients with abnormal conditions as compared to controls, N-Pyd levels measured in urine reflect the same increase in collagen degradation as do total H-Pyd levels measured after hydrolysis of the urine; and likewise, N-Dpd levels measured in urine reflect the same increase in collagen degradation as do total H-Pyd levels.

### V. Pre-Filtration of Urine Sample

In preferred embodiments of the method of assaying according to the present invention, a urine sample is filtered through a membrane which is effective to remove substance(s) that bind non-specifically to antibodies while allowing the N-Dpd or N-Pyd analyte to pass through. The filtered urine sample is reacted with a binding reagent (binding molecule) effective to form an antigen-antibody complex with the analyte, under conditions effective to bind the antibody to the solid support in proportion to the amount of analyte in the sample.

The method is based on the discovery that a substance(s) present in variable amounts in urine samples can interfere with the sensitivity, precision, and accuracy of immunoassays. The interfering substance varies from individual to individual, and can also vary in different samples obtained from the same individual.

In accordance with the invention, the interfering substance(s) can be removed by contacting a urine sample with a separation medium (e.g., filter membrane) which is characterized by (i) a binding capacity which is adequate for reducing the level of interfering substance(s) in the sample below a selected threshold amount, and (ii) a low affinity for the N-Dpd or N-Pyd analyte, i.e., such that the analyte concentration after sample treatment is essentially the same as before sample treatment. Other desirable features for the separation medium include rapidity of operation and a small retention volume.

Materials which may be used as separation media include nitrocellulose-based materials, hydrophobic/high protein-binding materials, and charged hydrophilic materials. The configuration of the medium can take a variety of forms, including a strip, bead, cartridge, filter disc, or spin filtration device for example. The efficacy of the separation medium in removing the interfering substance can be evaluated by methods described below.

In one preferred embodiment, illustrated in Example 13-14, the separation medium is configured as a spin filtration device which provides rapid sample through-put. Alternatively, the separation medium may be placed in the sample for a selected time to bind and thereby remove the interfering substance(s) from the sample. Studies carried out in support of the invention suggest that where the separation medium takes the form of a membrane, a small pore size (*e.g.*, less than about 0.45 µm, and preferably less than about 0.1 µm) can increase the binding capacity of the membrane for the interfering substance.

In general, it is useful initially to screen candidate separation materials based upon either low affinity for the N-Dpd or N-Pyd analyte or high affinity for the interfering substance(s).

Binding of analyte to a particular separation medium can be assessed using one or more standard solutions containing known levels of analyte. Preferably, non-urine solutions are used to avoid the effects of the interfering substance(s). The standard solutions are contacted with (e.g., filtered through) the separation medium, and the analyte is assayed to determine whether any analyte was bound to the separation medium. In another approach, the analyte-content of a urine sample which contains the interfering substance(s) is measured using an analytical method (e.g., HPLC) that is not affected by the interfering substance. The absence of change in the measured analyte level following contact of the sample with the separation medium indicates that further characterization of the medium is warranted.

capture of the interfering substance(s) by a particular separation medium can be assessed by a number of approaches. For example, the immunoassay which is used for measuring the analyte can be modified to provide an assay that reports the presence of the interfering substance regardless of the presence or absence of the analyte. As illustrated in Example 12, the interfering substance can be detected using an assay that measures the level of binding of reporter-labeled anti-antibody to the solid-support of a microtitre plate in the absence of analyte-specific antibody. A urine sample which causes such non-specific binding of anti-antibody to the solid-support can then be used to assess the effectiveness of a candidate separation medium to reduce or eliminate such non-specific binding caused by the sample.

Once a promising separation material has_been identified, the composition and binding capacity of the material can be optimized according to the requirements of the analyte immunoassay.

Typically, the analyte is assayed in a competitive format wherein the urine sample is reacted with an analyte-specific antibody reagent in the presence of a reporter-labeled competitor analyte; the amount of immunocomplex which is formed between antibody reagent and competitor analyte is related inversely to the amount of analyte present in the urine sample. Usually, either the analyte-specific antibody reagent or the reporter-labeled competitor analyte is immobilized on a solid support, as a surface-attached binding molecule. Alternatively, a sandwich assay format can be used, where a first antibody for binding analyte is immobilized on a solid support, and a second antibody which is labeled or can be labeled with a reporter (e.g., with an enzyme-labeled anti-antibody) is used to detect analyte which is bound to the first antibody reagent.

The effects of the interfering substance in a representative immunoassay are illustrated by the data shown in Table 6. These data were obtained using the immunoassay format illustrated in Fig. 1, with N-Pyd as analyte. As detailed in Example 11, aliquots of six urine samples before and after contact with a nitrocellulose filter were assayed, and the apparent level of analyte in each sample was determined by comparison with data obtained from a set of N-Pyd standard solutions.

**Table 6**

| **Quantitation of Pyd** | | | |
|---|---|---|---|
| **Urine ID** | **Pyd (nM) pre- filtration** | **Pyd (nM) pre- filtration** | **% Increase** |
| 2181 | 111 | 205 | 45.8 |
| 2176 | 167 | 201 | 16.9 |
| 2159 | 357 | 592 | 39.7 |
| 2167 | 337 | 365 | 7.7 |
| 2161 | 97 | 137 | 29.1 |
| 2172 | 244 | 362 | 32.6 |

As seen from Table 6, the measured concentration of N-Pyd increased by about 7-45 percent after removal of the interfering substance via nitrocellulose filter disc, indicating wide variability in the extent of interference among samples. Moreover, the analyte concentrations obtained with filtered samples correlated highly with N-Pyd concentrations in the sample, as measured by HPLC, indicating that sample variation due to the interfering substance is largely eliminated by contact with the separation medium.

In the study described in Example 12, the interfering substance is shown to mediate the binding of enzyme-labeled anti-antibody to the solid support in the absence of added anti-analyte antibody. Forty-four urine samples were assayed using the assay format for N-Pyd described in Example 8 in the absence of anti-analyte antibody. Of the 44 samples tested, eight produced optical density readings greater than 0.02 absorbance units, indicating the presence of a substance or substances mediating direct, non-Pyd-specific binding of the enzyme-labeled antibody to the solid support. This non-analyte-specific binding of antibody to the solid support is also observed with other reporter-labeled anti-antibodies, e.g., with rabbit anti-mouse antibodies.

Table 7 shows optical density measurements which were obtained with the 8 problematic urine samples from Example 12, before and after passage of the samples through a nitrocellulose membrane in a spin-filter device (Example 13). The table shows that without prior filtration, the urine samples tested gave rise to optical density readings ranging from 0.36 to 0.101 absorbance units. However, when the urine samples were spin-filtered through a nitrocellulose membrane, the optical density readings were decreased to less than 0.02 absorbance units for all of the samples. Thus, the nitrocellulose membrane is effective to remove the interfering substance such that non-specific binding of anti-antibody to the solid support is substantially reduced.

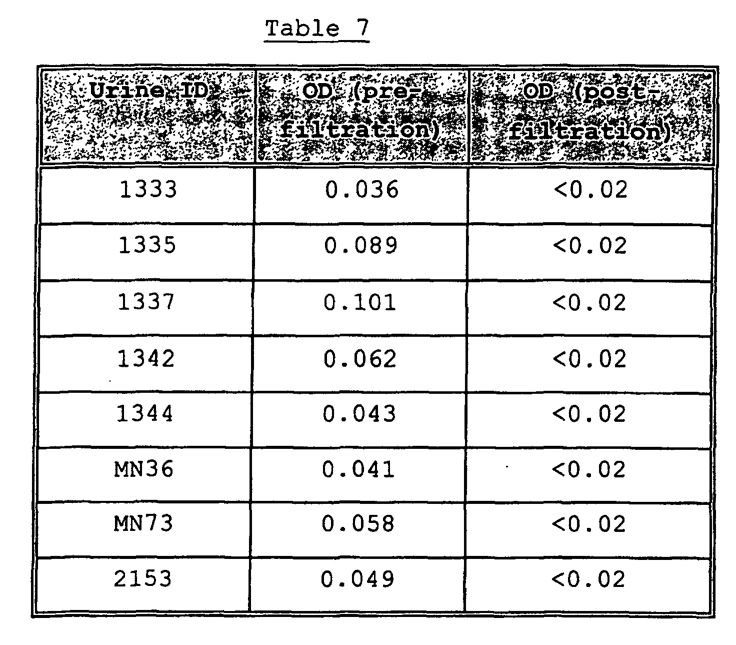

Tables 8 and 9 illustrate the comparable efficacies of three separation media that have been found useful in removing the interfering substance(s) from urine samples (Example 14). Table 8 shows optical density readings (OD) obtained with 16 urine samples before and after filtration. As can be seen, the optical density readings for the samples prior to filtration ranged from 0.0 to 0.905 absorbance units (second column). However, spin-filtration of the samples through Immobilon®-CD (CD) and Immobilon®-PSQ (PSQ) filters reduced the optical density readings of the samples to zero, similar to the results obtained with nitrocellulose (NC) (see columns 3-5).

**Table 8**

| **sample** | **OD (pre-filter)** | **OD (NC)** | **OD (PSQ)** | **OD (CD)** |
|---|---|---|---|---|
| 1 | 0.092 | 0.001 | -0.002 | -0.003 |
| 2 | 0.027 | -0.008 | 0.001 | 0.011 |
| 3 | 0.012 | -0.001 | -0.003 | 0.003 |
| 4 | 0.210 | -0.005 | -0.002 | 0.002 |
| 5 | 0.066 | -0.003 | 0.000 | -0.001 |
| 6 | 0.010 | -0.003 | 0.000 | 0.001 |
| 7 | 0.062 | -0.003 | 0.000 | -0.004 |
| 8 | 0.034 | -0.005 | -0.003 | -0.006 |
| 9 | 0.296 | -0.002 | -0.003 | -0.005 |
| 10 | 0.905 | 0.002 | 0.003 | 0.005 |
| 11 | 0.018 | 0.002 | -0.003 | 0.004 |
| 12 | 0.045 | 0.006 | 0.000 | 0.011 |
| 13 | 0.008 | 0.004 | 0.001 | 0.004 |
| 14 | 0.034 | -0.002 | 0.002 | 0.003 |
| 15 | 0.000 | -0.004 | 0.000 | -0.002 |
| 16 | 0.081 | 0.002 | -0.004 | -0.004 |

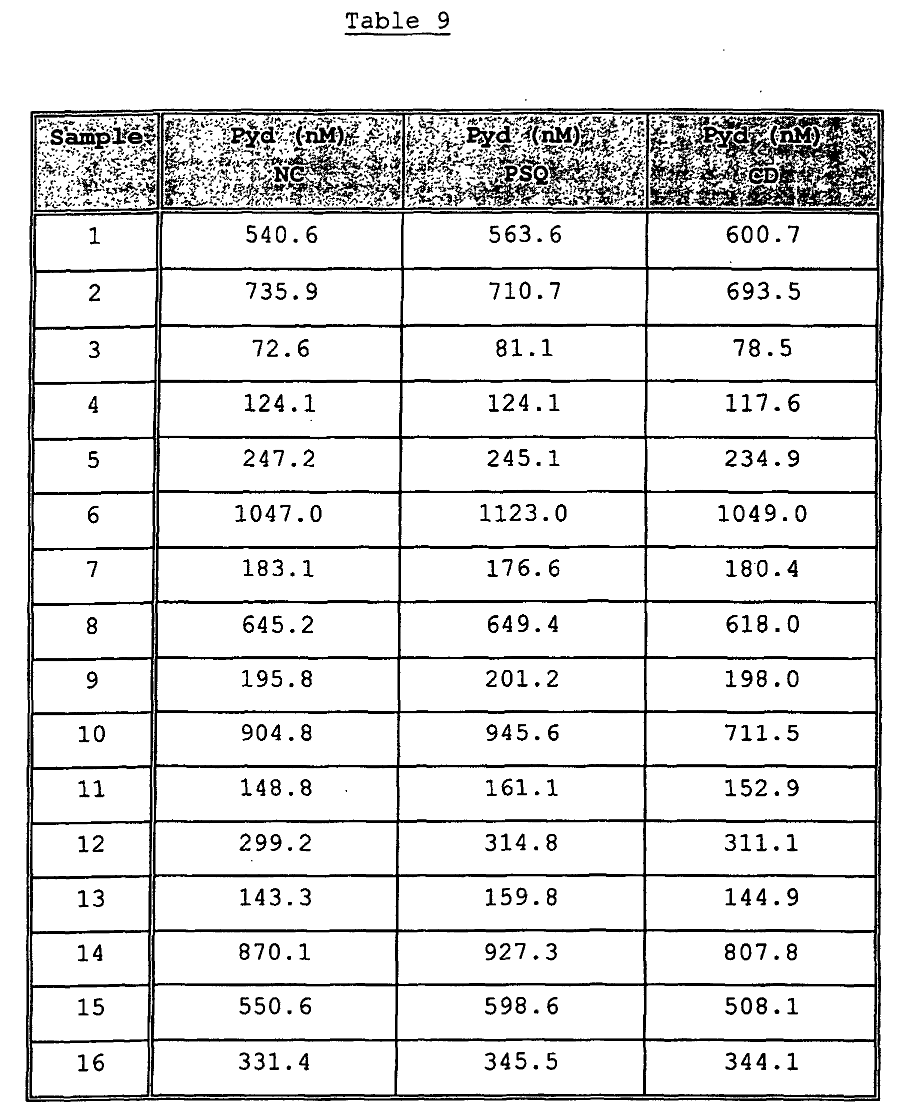

Table 9 shows that the levels of N-Pyd measured following filtration of the samples through the nitrocellulose (NC), Immobilon®-CD (CD), and Immobilon®-PSQ (PSQ) membranes were in excellent agreement with one another. Thus, all three media are suitable for removing the interfering substance.

### VI. Applications

As noted above, the pyridinium crosslink content of collagen-containing tissues varies according to tissue type. Pyd crosslinks are found in cartilage, bone, intervertebral discs, ligaments, and the aorta. Dpd crosslinks are generally less prevalent than Pyd crosslinks and are found in bone, dentine, ligaments, and the aorta. The proportion of Dpd in tissue pyridinium crosslinks appears to be highest in bone, which has a Pyd:Dpd ratio of between 3:1 and 4:1.

Conditions which are characterized by increased urinary Dpd (and Pyd) levels, and thus are accompanied by elevated rates of bone degradation, include osteoporosis, Paget's disease, hypothyroidism, and osteoarthritis, for example. Other conditions involving increased Pyd and Dpd levels include various forms of metastatic cancer which alter bone metabolism or become established in bone tissue.

The present invention is based in part on the discovery that elevated urinary levels of N-Pyd and N-Dpd are reliable indicators of increased collagen degradation activity in humans. Measurement of an elevated level of N-Pyd or of both N-Pyd and N-Dpd serves as a general indicator of increased collagen degradation. Measurement of urinary N-Dpd via a Dpd-specific antibody in accordance with the invention serves as a relatively specific marker for elevated degradation of bone.

The invention is also useful in monitoring antiresorptive therapies associated with management of conditions described above.

In addition, where an assay for N-Pyd is used in combination with an assay for N-Dpd, the invention is useful for distinguishing conditions involving the breakdown of collagen-containing tissues in which the proportion of Dpd crosslinks is substantially less (relative to Pyd crosslinks) than the proportion found in bone. In rheumatoid arthritis, for example, which appears to involve breakdown of connective tissues relatively low in Dpd crosslinks, the ratio of urinary N-Pyd to N-Dpd is elevated compared to the ratio observed with increased bone degradation alone.

Fig. 3 illustrates use of a Pyd-specific antibody reagent in the assay configuration shown in Figs. 1A-1C, for detecting elevated N-Pyd levels in urine samples from metastatic cancer patients. The column of points shown on the left of the figure are levels of N-Pyd (expressed as Pyd/creatinine ratios) measured in a group of healthy subjects. The column of points on the right of the figure are N-Pyd levels measured in patients diagnosed with metastatic cancer. The data reveal patients who have elevated N-Pyd levels, indicating likely bone involvement in their oncological conditions.

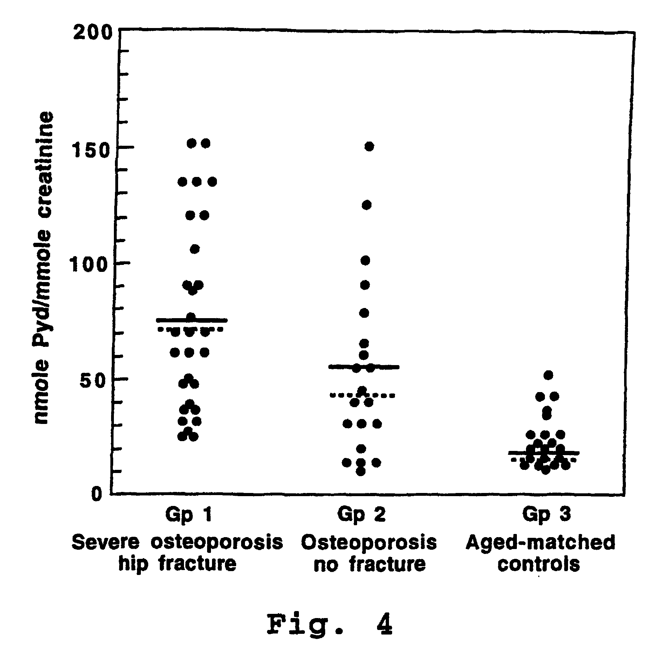

Fig. 4 illustrates use of a Pyd-specific monoclonal antibody reagent for detecting elevated N-Pyd levels in urine samples from osteoporosis patients. The column of points on the right of the figure (group 3) are N-Pyd levels (expressed as Pyd/creatinine ratios) measured in a group of 27 control subjects (age 70-90 years). The middle column of points are N-Pyd levels measured in 20 patients (aged 71-99 years) with overt or suspected osteoporosis (group 2). The column of points on the left (group 1) are N-Pyd levels measured in 30 patients with femoral neck fractures associated with severe osteoporosis. The data show elevated levels of urinary N-Pyd in both osteoporosis patient groups (2 and 3) as compared to the age-matched control group (group 1).

Use of a Dpd-specific monoclonal antibody in accordance with the invention, for detecting elevated levels of urinary Dpd, is illustrated in Fig. 5. In this study, the assay configuration shown in Figs. 1A-1C was used, adapted to detection of N-Dpd. The points in the left-hand column in the figure are measured Dpd levels (expressed as Dpd/creatinine ratios) for a group of healthy patients. The points in the center column in the figure are the measured Dpd levels from a patient population which includes patients with diagnosed bone metabolism disorders. The right-hand column of points in the figure are measured Dpd levels for a group of oncology patients. The test identifies those patients with elevated Dpd levels, i.e., those patients whose oncological condition likely includes bone involvement.

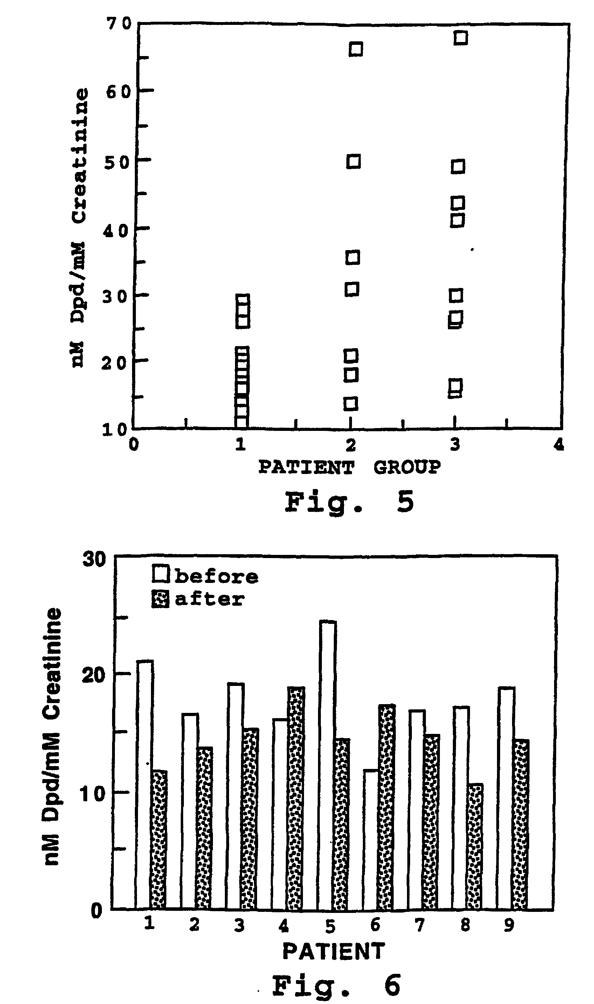

Figure 6 illustrates the use of a Dpd-specific monoclonal antibody of the invention for monitoring estrogen therapy in women under treatment for osteoporosis. In this study, Dpd levels were measured prior to therapy (solid bars in the figure) and after 1 year of estrogen therapy (shaded bars). In seven of the patients, a measurable drop in Dpd levels was observed with therapy, indicating that the therapy is producing the desired reduction in bone resorption. The results in the latter two cases, where Dpd levels have increased with therapy, may indicate that alternative treatment should be considered.

From the foregoing, it can be appreciated how the objects of the invention are met. The native free crosslink assay can be used with a non-hydrolyzed urine sample, thereby avoiding the need for a preliminary acid hydrolysis step. The assay utilizes an antibody reagent, and can thus be adapted to a number of convenient and rapid assay formats known in the art. Moreover, by providing an assessment of the level of native free Pyd, Dpd, or both, in a urine sample, the invention can be used to detect and monitor a variety of collagen-related pathology states.

The following examples illustrate methods of producing antibody reagents and assay methods, in accordance with the invention. The examples are intended to illustrate, but in no way limit, the scope of the invention.

### EXAMPLES

### Materials and Methods

Glucose-6-phosphate dehydrogenase (G6PDH, from Leuconostoc mesenteroides; å (280 nm, 1%) = 1.15 ml/mg.cm; suspension in ammonium sulfate), glucose-6-phosphate (G6P), and nicotinamide adenine dinucleotide (NAD) were purchased from Boehringer Mannheim. 5,5'-Dithiobis(2-nitrobenzoic acid) (DTNB), cysteine hydrochloride, N-succinimidyl S-acetylthioacetate (SATA), sulfosuccinimidyl 4-(N-maleimidomethyl)cyclohexane-1-carboxylate (sulfo-SMCC), 1-ethyl-3-(3-dimethylamino-propyl)carbodiimide-HCl (EDC), and N-succinimidyl 3-(2-pyridyldithio)propionate (SPDP) were purchased from Pierce (Rockford, IL). *p*-Nitrophenylphosphate tablets (20 mg each), N-ethyl maleimide (NEM), and diethanolamine were purchased from Sigma. Dialysis tubing was purchased from Spectrum Laboratories. Microtiter plates (96-well) were purchased from Nunc and from Costar Corp. Absorbance values of solutions in microtiter plates were determined using a V-Max microplate reader from Molecular Devices Corp. (Menlo Park, CA). Slow, dropwise additions of reagents was accomplished using a Microperplex peristaltic pump #2132 from LKB.

### A. Reagents for Monoclonal Antibodies

Female autoimmune MRL/MpJ-lpr mice were purchased from the Jackson Laboratory, Bar Harbor, Maine.

Mouse non-secreting P3X63Ag8.653 myeloma cells, and mouse monocyte-macrophage cell lines P388D1(IL-1) and J774A.1 were purchased from American Type Culture Collection (ATCC), Rockville, Maryland.

Adjuvant Ribi and Ribi(CWS) were purchased from RIBI Immunochem Research, Inc., Hamilton, Montana. 50% PEG 1500 (polyethylene glycol 1500, 50% (w:v) in water) was purchased from Boehringer Mannheim, Indianapolis, Indiana. HAT and HT were purchased from Sigma Chemical Company, St. Louis, Missouri.

Dulbecco's Modified Eagle Medium (DMEM), NCTC-109, and gentamicin were purchased from Gibco, Grand Island, New York.

FetalClone bovine serum was from Hyclone Laboratories, Inc., Logan, Utah. Oxaloacetic acid and insulin were from Sigma Chemical Company. S-DMEM was formulated as follows, where the percentages indicate final volume percentages in the final medium: DMEM (80%), NCTC-109 (10%), FetalClone bovine serum (10%), oxaloacetic acid (1 mM), L-glutamine (2 mM), gentamicin (50 ìg/ml) and insulin (10 ig/ml).

For preparation of conditioned media, mouse monocyte cell lines P388D1 (IL-1), or interchangeably, cell line J774A.1, were grown in S-DMEM medium, with a 1:4 split twice a week. Every 3 days, tissue culture supernatants were filtered through a 0.2 micron filter and then supplemented with 4 mM L-glutamine. The resultant concentrated conditioned media were used as 20% supplement for S-DMEM to raise hybridoma cells.

Unless stated otherwise, PBS is defined as a buffer containing 0.01 M phosphate and 150 mM NaCl, pH 7.

### Example 1

### HPLC Measurement of Crosslinks

HPLC analysis for Pyd and Dpd was done essentially as described by Black et al. (1988). Briefly, urine samples were adjusted with butanol and glacial acetic acid to produce a 4:1:1 mixture (butanol:acetic acid:sample, v:v:v) and applied onto CF1 cellulose (Whatman) cartridges, followed by a wash with 4:1:1 butanol:acetic acid:water. Only free crosslinks (and glyco-Pyd) were retained. The free crosslinks were eluted from CF1 cellulose with water. Eluted material was analyzed on a C18 reverse phase column (Rainin, C18-80-200-C3) using a water-acetonitrile (3-17% in 10 minutes) gradient delivered at 1 ml/minute and monitoring fluorescence at 295 nm of excitation, 395 nm of emission. Mobile phase contained 0.1% HFBA.

Total urinary crosslinks were measured by hydrolyzing a urine sample in HCl (6N) at 110°C for 16 hours, followed by the CF1 pretreatment and HPLC analysis as above. HPLC separation yielded hydrolysed Pyd and Dpd fractions, from which total H-Pyd and total H-Dpd were quantitated.

### Example 2

### Purification of Crosslinks

Human urine was filtered through 3000 D molecular cut-off filter (Filtron Co.) applying 40 psi of back pressure. The filtrate was then lyophilized and reconstituted to 1/20 of the original volume with 0.2 M acetic acid.

Concentrated urine was then applied onto a Sephadex®G-10 2.6 × 95 cm column equilibrated with 0.2 M acetic acid. Material eluted from the column was analyzed for free Pyd and Dpd as described above. The free crosslink-containing fractions were pooled together, adjusted to pH 2.0 and applied onto a 1 × 18 cm cation exchange column (Lacarte Co., UK) equilibrated with 0.1 M sodium citrate, pH 4.2.

Glyco-Pyd, N-Pyd and N-Dpd were coeluted thereafter from the column with 0.1 M sodium citrate pH 4.2. Collected fractions were analyzed for the presence of crosslinks by HPLC analysis as above. Fractions containing specific crosslinks (glyco-Pyd, Pyd and Dpd) were pooled together and applied onto a preparative 2.5 × 10 cm reverse phase C18 column (Waters) which was subsequently developed with a 2-20% gradient of acetonitrile containing 0.1% HFBA. Separated fractions (N-Pyd and N-Dpd) were collected separately and concentrated by lyophilization. The dry residues were reconstituted in 0.2 M acetic acid and stored at 4°C. Purity of the final materials was measured by gravimetric and elemental analysis.

Urinary crosslink-peptides were prepared by exhaustive dialysis of human urine using 1000 D molecular weight cut-off dialysis membranes (Spectra/Por®). The total Pyd crosslink and total Dpd crosslink contents of the peptide fractions was determined by hydrolyzing peptide samples with 6N HCl at 110°C for 16 hours followed by HPLC analysis for total H-Pyd and total H-Dpd.

Preparative amounts of H-Pyd and H-Dpd were obtained from hydrolyzed powdered bovine or sheep bone as described by Black *et al*. (1988).

### Example 3

### Preparation of Immunogens

The following procedures illustrate how immunogens can be prepared for obtaining monoclonal or polyclonal antibodies against native free pyridinoline, native free deoxypyridinoline, or both. The procedures in A and B below are described with respect to H-Pyd-immunogens; H-Dpd-immunogens are prepared using the same approach, with H-Dpd instead of H-Pyd.

### A. H-Pyd-BSA Immunogen

To-a 3.1 ml solution consisting of 9 mg of bovine serum albumin (BSA) and 3.8 mg of H-Pyd in 0.1 M MES pH 5.0 was added a 0.88 ml aqueous solution containing 88 mg of EDC. The mixture reacted for four hours at room temperature then was exhaustively dialyzed versus phosphate buffered saline pH 7.0 (PBS). UV and fluorescence measurements indicated 5.8 moles of pyridinoline substituted per mole of albumin.

### B. H-Pyd-KLH Immunogen

To a solution of dried H-Pyd (6 mg) in water adjusted to pH 5 ± 0.5 (200 il) was added 2 ml of a 10 mg/ml solution of keyhole limpet hemocyanin (KLH) in PBS. To the mixture was added 30 mg solid 1-(3-dimethylaminopropyl)-3-ethylcarbodiimide (EDC, Pierce), and ten minutes later, another 30 mg of EDC, and the reaction was allowed to proceed for 4 h at room temperature. The reaction mixture was then exhaustively dialyzed versus PBS, after which the H-Pyd-KLH immunogen was collected and stored.

### Example 4

### Preparation of Anti-Pyd Monoclonal Antibodies

### A. Immunization Protocol

Female 5-week-old autoimmune MRL/MpJ-lpr mice were immunized using the protocol below:

**Table 10**

| **Immunization Protocol** | | | | |
|---|---|---|---|---|
| **Immunization** | **Days from Fusion** | **Immunogen Injected (µg)** | ^{**1**}**Adjuvant** | **Inject. Mode** |
| 1 | 60 | 100 | Ribi | ip² |
| 2 | 46 | 100 | Ribi | ip |
| 3 | 32 | 100 | Ribi | ip |
| 4 | 18 | 100 | Ribi | ip |
| 5 | 4 | 200 | -- | iv³ |

| | | | | |
|---|---|---|---|---|
| ¹Adjuvant and antigen were suspended in Hank's balanced salt solution | | | | |
| ²Intraperitoneal | | | | |
| ³Intravenous | | | | |

On the day of fusion, the immunized mouse was sacrificed by CO₂ gas, and the spleen was excised from the mouse and placed in a culture dish containing 5 ml of serum-free DMEM medium preheated to 37°C. Following removal of adipose tissue attached to the spleen, the spleen was washed with 5 ml of serum-free DMEM medium. The spleen was then cut into small pieces which were placed in a cell homogenizer containing 7 ml of serum-free DMEM medium, and the cells were homogenized to form a cell suspension.

### B. Fusion Protocol

The following steps were performed at room temperature.

The spleen cell suspension (∼2 × 10⁸ cells in serum-free DMEM medium) and log-phase P3X63Ag8.653 myeloma cells (∼7 × 10⁷ cells in serum-free DMEM medium) were centrifuged independently at 400xg for 10 min. The resultant cell pellets were suspended together in serum-free DMEM medium (10 ml) in a 50 mL centrifuge tube and then centrifuged at 400xg for 10 min. The supernatant was removed completely, and the centrifuge tube was tapped to loosen the cell pellet.

For cell fusion, a solution of 50% PEG 1500 (4 ml) was added dropwise to the tube with gentle mixing by pipette over a 90 second period. Next, serum-free DMEM (4 ml) was added dropwise over 1 min. S-DMEM (40 ml) was then added over 2 min with gentle mixing, after which the mixture was mixed by pipette for an additional 2.5 min. The resultant mixture was centrifuged at 400xg for 10 min. After thorough removal of the supernatant, the cells were suspended in 320 ml of HAT in 20% P388D1-conditioned S-DMEM medium. The cell suspension was plated in 16 96-well tissue culture plates, 200 µl/well, and the plates were then incubated at 37°C in an atmosphere containing 7% CO₂. The cell mixtures were fed at day 3 and day 7 by removing 100 µl/well of old medium and adding 150 µl/well of either HAT medium (day 3) or HT medium (day 7). The wells were ready to screen 7 to 10 days after fusion.

### C. Screening Hybridomas for Production of Anti-N-Pyd Monoclonal Antibodies

Successful fusion products were screened for immunoreactivity using the N-Pyd immunoassay format described in Example 8. Cell lines which showed high affinity binding to N-Pyd were subcloned by limiting dilution and further screened for production of antibodies with high binding affinity for N-Pyd. One of the subcloned cell lines which gave high antibody affinity for N-Pyd is designated herein as Mab Pyd-XXV-3G6-3B11-1A10. The specificity of antibodies produced by this cell line is shown in Table 1 above.

### Example 5

### Preparation of Anti-Dpd Monoclonal Antibodies

Anti-Dpd monoclonal antibodies were prepared by the procedure described in Example 4, using H-Dpd-KLH inmunogen prepared as in Example 3. The mouse immunization procedure was the same as in Example 4, except that Ribi(CWS) was used as adjuvant instead of Ribi, and 75 µg immunogen per mouse was used in the fourth immunization step (18 days from fusion) instead of 100 µg.

Successful fusion products were screened for immunoreactivity using the N-Dpd immunoassay format described in Example 9. Cell lines which showed high affinity binding to N-Dpd were subcloned by limiting dilution and further screened for production of antibodies with high binding affinity for N-Dpd. One of the subcloned cell lines which gave high antibody affinity for N-Dpd is designated herein as Mab Dpd-II-7B6-1F4-1H11. The specificity of antibodies produced by this cell line is shown in Table 2 above.

### Example 6

### Preparation of Monoclonal Antibodies Specific for Both N-Pyd and N-Dpd

Monoclonal antibodies specific for both N-Pyd and N-Dpd were prepared by the procedure in Example 5, using H-Dpd-KLH (Example 3) as immunogen. Successful fusion products were screened for immunoreactivity using the N-Dpd immunoassay format described in Example 9. Cell lines which showed high affinity binding to N-Dpd were subcloned by limiting dilution and further screened for production of antibodies with high binding affinity for both N-Pyd and N-Dpd. One of the subcloned cell lines which gave high antibody affinity for both N-Pyd and N-Dpd is designated herein as Mab Pyd/Dpd-V-6H2-2H4-1E4. The specificity of antibodies produced by this cell line is shown in Table 3 above.

### Example 7

### Preparation of H-Pyd-Streptavidin and H-Dpd Streptavidin

Conjugation of H-Pyd to streptavidin was accomplished by coupling a thiolated streptavidin to H-Pyd via the coupling agent, SMCC. Thiolated streptavidin was prepared by reaction with N-succinimidyl-3-(2-pyridyldithio)propionate (SPDP, Pierce) as follows. To a 0.75 ml solution of 5 mg of streptavidin in PBS was added 21 uL of dimethylformamide containing 260 ug of SPDP. The mixture was allowed to react for one hour at room temperature, and then was dialyzed against PBS. The SPDP-labeled streptavidin was reduced by the addition of dithiothreitol to a final concentration of 10 mM. After incubation for one hour at room temperature, the thiolated streptavidin was purified on a G-25 column.

To form H-Pyd-streptavidin, a solution containing 180 ug of succinimidyl 4-(N-maleimidomethyl)cyclohexane-1-carboxylate (SMCC, Pierce) in dimethylformamide (4 ul) was added to a solution containing 0.5 mg thiolated streptavidin and 50 ug of H-Pyd in 100 ìl of PBS. The mixture was allowed to react for 3 hours at room temperature and then was dialyzed versus PBS. Spectrophotometric analysis of the resultant Pyd-streptavidin indicated between 1 and 2 equivalents of pyridinoline bound per equivalent of streptavidin.

H-Dpd-streptavidin was prepared by the same procedure except that H-Dpd was used in place of H-Pyd.

### Example 8

### Immunoassay Using Anti-Pyd Monoclonal Antibody Reagent

### A. H-Pyd-Coated Microtitre Plates

Biotin-labeled ovalbumin and H-Pyd-streptavidin (Example 7) were utilized in the microplate coating.

Biotinylation of ovalbumin was carried out by adding 10 mg of biotin-X 2,4-dinitrophenyl-X-L lysine, succinimidyl ester (Molecular Probes) in 400 µl of dimethylformamide to a 10 ml solution of PBS containing 150 mg of ovalbumin. The mixture was reacted for two hours at room temperature followed by G25 column chromatography. Spectrophotometric analysis indicated two biotins substituted per mole of ovalbumin.

The wells in a 96-well ELISA plate were coated with N-Pyd as follows. To each well was added 150 µl of biotin-ovalbumin solution at 3.8 ug/ml in PBS, followed by an overnight incubation at 2-8°C. The wells were washed with PBS and blocked by adding 200 ul of ovalbumin at 1 mg/ml with an overnight incubation at room temperature. The wells were then twice washed with PBS. 150 ul of a solution containing H-Pyd-streptavidin at 100 ug/ml in PBS was added to each well of the biotin-ovalbumin coated microplate. After a one hour incubation at room temperature, the wells were twice washed with PBS. Residual liquid was removed from the wells by drying overnight in a convection oven at 37°C.

### B. Assay Protocol

N-Pyd standard solutions and urine samples were tested in duplicate. The standard solutions consisted of 0 nM, 25 nM, 75 nM, 250 nM, 750 nM, and 3000 nM N-Pyd in assay buffer (0.05% NaN3, 0.05% Tween 20, and 0.1% BSA in PBS). Urine samples were filtered through a nitrocellulose membrane by centrifugation (spin filtration) prior to assay.

Using microtitre plates prepared as above, 20 ul/well of the standard solutions were added in duplicate to 12 of the 96 wells in the coated plates, and 20 ul/well of each of 44 urine samples were added in duplicate to wells of two microtitre plates.

100 ul/well of MAb 3G6-3B11-1A10 (1:100,000 dilution) in assay buffer (0.05% NaN3, 0.05% Tween 20, and 0.1% BSA in PBS) were added. The standards or samples and MAb mixtures were incubated at 4°C overnight. 100 ìl/well of goat anti-mouse IgG + M(H+L)-alkaline phosphatase conjugate (Pierce, No. 31330, 1:1000 dilution in assay buffer) were added to the plate and incubated at room temperature for one hour.

To each well was added 100 uL of enzyme substrate solution (2 mg/mL of p-nitrophenylphosphate (Sigma) in 1.0 M diethanolamine, pH 9.8, containing 1 mM MgCl₂). Following a 30 min incubation at room temperature, 50 il of 3.0 N NaOH was added to each well to stop the enzymatic reaction. The optical density at 405 nm was then measured with a Vmax reader (Molecular Devices Corp.).

The optical density readings (405 nm) from duplicate samples were averaged, and the averaged readings from the N-Pyd standards were used to construct a standard curve of OD reading vs. N-Pyd concentration. From this curve, the free N-Pyd crosslink concentration in each urine sample was determined. Creatinine concentrations were measured using an assay involving alkaline picrate (Cook, 1975).

The same urine samples were quantitated for total Pyd by the HPLC method described in Example 2. Briefly, the urine samples were hydrolysed in HCl (6N) at 110°C for 16 hours, followed by the CF1 pretreatment and HPLC analysis for total H-Pyd.

Fig. 2 shows a scatter plot of pyridinium crosslinks (in nM) measured by the immunoassay method (y-axis) measured vs. total H-Pyd measured by HPLC. The line in the plot is the best-fit linear regression equation which correlates immunoassay values as a function of HPLC total Pyd values.

### Example 9

### Immunoassay Using Anti-Dpd Monoclonal Antibody Reagent

### A. H-Dpd-Coated Microtitre Plate

To each well of a 96-well ELISA plate was added 100 µl of a solution containing H-Dpd-streptavidin (0.5 µg/ml in PBS). After overnight incubation at 4°C, the wells were emptied and then blocked by incubation with ovalbumin (1 mg/ml in PBS, 150 µl/well) for at least 1 h at room temperature or overnight at 4°C. After incubation, the plates were washed 3 times with PBS.

### B. Assay Protocol

N-Dpd standard solutions and urine samples were tested in duplicate. The standard solutions consisted of 0 nM, 25 nM, 50 nM, 100 nM, 250 nM, and 500 nM N-Dpd in assay buffer (0.05% NaN3, 0.05% Tween 20, and 0.1% BSA in PBS). Urine samples were filtered through a nitrocellulose membrane by centrifugation (spin filtration) prior to assay.

Following the addition of sample (10 µl/well), 100 µl/well of 7B6-1F4-1H11 monoclonal antibody (1:1600 dilution of tissue culture supernatant into assay buffer, ∼10 ng/ml antibody) was added, and the assay plate was incubated at 4°C overnight. After the plate was washed 3 times with 300 µl/well of wash buffer, 100 µl/well of goat anti-mouse IgG+M(H+L)-alkaline phosphatase conjugate (Pierce, No. 31330) (1:1000 dilution in assay buffer) was added, and the plate was incubated at room temperature for 1 h. The wells were then washed 3 times with wash buffer.

Bound enzyme was assayed as in the preceding Example. The N-Dpd crosslink concentration for each unknown urine sample was determined by quantitation from the standard curve.

### Example 10

### Binding Selectivity of Monoclonal Antibody Reagents

N-Pyd, N-Dpd, pyridinium-peptides (MW>1000) were isolated from urine samples as described above. Aliquots of the pyridinium preparations were hydrolysed to convert the crosslinks in the fractions to H-Pyd and H-Dpd. The concentrations of Pyd in the N-Pyd and H-Pyd preparations, of Dpd in the N-Dpd and H-Dpd preparations, and of Dpd in the pyridinium-peptide preparations, were determined by HPLC, as in Example 1. In addition, an amino acid solution containing an equimolar mixture of the 20 common amino acids, 150 µM each in PBS, was prepared.

Aliquots (50 µl) of the native crosslink preparations and the amino acid mixture were added in duplicate to Pyd-coated or DpD-coated microtitre wells, prepared as above, and each well was assayed for pyridinoline or deoxypyridinoline as in Example 8 or 9, as appropriate. The optical density readings (405 nm) from duplicate samples were averaged, and from these values, the apparent N-Pyd or N-Dpd concentration of each sample was determined using a standard curve established with purified N-Pyd or N-Dpd. The percent reactivity of each sample was calculated as a ratio of apparent concentration (using a standard curve; see Examples 8 and 9) to total Pyd crosslink concentration in the sample determined by HPLC for total H-Pyd (times 100), or total H-Dpd in the case of total Dpd crosslink concentration. Results obtained with antibodies having various specificities are given in Tables 1, 2 and 3 above.

### Example 11

### Nitrocellulose Syringe Filtration

Six urine samples designated 2181, 2176, 2159, 2167, 2161, and 2172 were tested. An aliquot (400 il) of each sample was syringe-filtered through a 25 mm diameter 0.45 im pore size nitrocellulose filter (Millipore, Millex-HA, Bedford, MA) and the filtrate was collected in a tube. Aliquots (10 ìl) of filtered and unfiltered sample material and of N-Pyd standards were added in duplicate to the wells of a 96 well microtitre plate prepared as in Example 8, and the concentration of N-Pyd in each sample was determined as generally described in Example 8, using an N-Pyd-selective polyclonal antibody, and an alkaline phosphatase-labeled goat anti-rabbit antibody. The results are shown in Table 6.

### Example 12

### Assessment of Non-Specific Binding Mediated by Urine Samples

Urine samples were tested for the presence of interfering materials by the following procedure. Aliquots (10 ìl) of buffer, N-Pyd standards, and each of 44 urine samples were placed in duplicate in the wells of two 96-well microtitre plates. The assay procedure described in Example 8 was carried out except that the anti-N-Pyd antibody was replaced with assay buffer or with diluted tissue culture supernatant. Wells to which buffer alone, diluted tissue culture, or N-Pyd standard had been added afforded optical density readings of less than 0.010. Of the 44 urine samples tested, 8 produced readings which were greater than 0.02 absorbance units (Table 7).

### Example 13

### Removal of Interfering Substance by Spin Filtration

The 8 urine samples from Example 12 which produced optical density readings greater than 0.02 were subjected to a spin filtration step as follows. An aliquot (200 ìl) of each sample was placed in a nitrocellulose filter unit suspended inside a 1.5 mL microfuge tube (the filter unit and microfuge together are referred to as "filter assembly"). The filter unit (constructed by Lida Manufacturing Corp, Kenosha, WI) included a polypropylene cylindrical housing (40 mm length × 10 mm outer diameter) containing at its lower end a double layer of nitrocellulose membranes (0.1 im pore size) and a non-woven polyethylene support. The effective filtration cross-section of the nitrocellulose membranes was 0.2 cm². The sample-containing filter assemblies were then centrifuged at 1500 × g for 2 minutes to pass the samples completely through the filters. The samples were then processed as described in Example 8, but with assay buffer used in place of anti-Pyd antibody. The measured optical densities of all eight filtered samples were less than 0.02 (Table 7).

### Example 14

### Comparison of Spin Filters

Aliquots (200 ìl) of 16 urine samples were centrifuged in spin-filter assemblies containing one of the following membrane materials: nitrocellose (Lida Manuf. Corp, Kenosha, WI), Immobilon®-CD (Millipore Corp., Bedford, MA, catalog no. ICDM-00000), and Immobilon®-PSQ (Millipore, catalog no. ISEQ-00000). The nitrocellulose-containing filter assembly was configured as described in Example 13. The Immobilon®-containing filter assemblies were configured similarly to those which contained nitrocellulose, except that the Immobilon® membranes were single- rather than double-layered, with a 0.1 µm pore size.

Aliquots (10 µl) of filtered and unfiltered urine samples were added in duplicate to the wells of a 96 well microtitre plate prepared as in Example 8. The assay procedure described in Example 8 was carried out except that the anti-N-Pyd antibody was replaced with assay buffer. The optical density readings produced by the urine samples are shown in Table 8 (all values were corrected by subtracting the absorbance of buffer alone). N-Pyd concentrations measured in the same samples using a calibration curve established with N-Pyd standards are shown in Table 9.

### Example 15

### Preparation of Pyridinoline-G6PDH Conjugate Using a Carbodiimide

Precipitated G6PDH (110,000 MW) from an ammonium sulfate precipitate (Boehringer Mannheim) was collected by centrifugation at 12,000 rpm for 5 min. To each of a number of aliquots of the pellet was added a solution of hydrolyzed Pyd or Dpd (15, 23, or 37 mM stock solutions) in 0.1 M phosphate containing 150 mM NaCl (PBS), pH 7.5, at a molar ratio (Pyd or Dpd to G6PDH) of 80:1, 130:1, or 200:1), and the resultant mixture was mixed. The final concentration of G6PDH in the mixture was 20 mg/ml.

Following addition of a selected amount of dry EDC to the solution (EDC to G6PDH molar ratio of 170:1, 290:1 or 460:1), the resultant mixture was allowed to react overnight at 4°C. The resultant Pyd-labeled G6PDH from each reaction mixture was purified on a Sephadex® G-25 column using 0.01 M PBS, pH 7.0, as elution buffer, or by dialysis against 0.01 M PBS, pH 7.0. The ratio of Pyd to G6PDH in each conjugate was calculated based on absorbance values at 280 nm (G6PDH, ε(280 nm, 1%) = 1.15 ml/mg·cm) and 326 nm (Pyd, ε(326 nm) = 5420 ml/mmol·cm, pH 7; or Dpd ε(326 nm) = 4933 ml/mmol·cm, pH 7). The conjugate was stored at -20°C in 50 % glycerol. G6PDH, Pyd, and EDC in the proportions 1:130:290 afforded G6PDH labeled with about 8.8 Pyd molecules per molecule of G6PDH.

### Example 16

### Preparation of Pyridinoline-G6PDH Conjugate Using Sulfo-SMCC and SATA

Thiolated G6PDH was prepared as follows. A solution containing G6PDH (2 mg/mL) and 5 molar equivalents of NAD⁺ was prepared in 0.1 M PBS, pH 7.5. To this solution was added 25 molar equivalents of SATA dissolved in (10.5 µL) anhydrous dimethyl sulfoxide. After a reaction period of 45 minutes at room temperature, 30 molar equivalents of NH₂OH·HCl was added, and the resultant mixture was allowed to react for 30 minutes to deprotect the thiol groups of the enzyme-bound SATA molecules. The resultant thiolated G6PDH was purified from residual MH₂OH·HCl and unbound SATA by centrifugation at 900 rpm through a PD-10 gel filtration column (Pharmacia).

A 1:1 Pyd:SMCC derivative was prepared as follows. To a vigorously stirred solution of hydrolyzed Pyd (29 mM) in 0.1 M PBS, pH 7.5, was added slowly a solution containing 1.5 molar equivalents of sulfo-SMCC in 200 µL of a 1:1 mixture of MeOH and 0.1 M PBS, pH 7.5. The resultant clear, dark amber solution was stirred for an additional 3 hrs. Following storage of the reaction solution at -20°C for 2 days, the solution was passed through a column of BioGel P-2 (BioRad) using 10 mM PBS, pH 7.5, as eluent. Three Pyd-containing peaks were observed by UV monitoring at 326 nm: Pyd:SMCC (1:2); Pyd:SMCC (1:1); and free (underivatized) Pyd. Fractions containing the 1:1 Pyd:SMCC derivative were pooled, and the concentration was determined based on absorbance of the Pyd pyridinium ring at 326 nm.

Pyd-labeled G6PDH was prepared by addition of 1.3 mL of thiolated G6PDH solution (0.8 mg/mL, prepared as above) with 63 ìL of a 1.7 mM solution of the 1:1 Pyd:SMCC derivative (12 molar equivalents of Pyd:SMCC derivative per equivalent of G6PDH). The pH of the resultant mixture was adjusted to 7.5 if necessary, and the mixture was allowed to react for 1 hr at room temperature sealed under an argon atmosphere.

Any remaining thiols were capped by reaction with a methanolic maleimide solution (1 M stock, final concentration, 50 mM) for 1 hr. Following this capping step, the resultant mixture was dialyzed overnight in 12-14,000 MW cut-off dialysis tubing in 10 mM PBS, pH 7, at 4°C in the dark. After dialysis, the final volume was measured and the ratio of G6PDH:Pyd was calculated based on the absorbances at 280 and 326 nm.

### Example 17

### Preparation of Pyridinoline-G6PDH Conjugate Using sulfo-SMCC and SPDP

The following buffers were prepared and degassed: 0.05 M sodium phosphate, pH 7.5, containing 115 µM NAD⁺ (pH 7.5 buffer); 0.10 M sodium phosphate, pH 6.0, containing 115 µM NAD⁺ (pH 6 buffer).

To a vigorously stirring solution of G6PDH (2 mg/mL, 0.5 mL) in pH 7.5 buffer was added 14 or 47 µl of a 19 mM solution of SPDP (30 or 100 molar equivalents) in anhydrous ethanol over 1-2 hrs using a peristaltic pump. When the addition had been completed, the resulting solution was dialyzed exhaustively using 6-8,000 or 12-14,000 MW cut-off tubing against pH 6 buffer at 4°C. Following dialysis, the SPDP-labeled enzyme was collected and reacted with dithiothreitol (5-10 mM for 30 minutes) to remove the 2-thiopyridine groups, thereby producing a thiolated enzyme. The number of SPDP molecules bound to enzyme was determined by measuring the amount of pyridine 2-thione (A₃₄₄) released in the dithiothreitol reaction. The resultant thiolated enzyme was purified on a PD-10 desalting column with pH 6 buffer, and the actual thiol content of the enzyme (5 and 10 thiol groups per molecule of G6PDH, obtained with 30 and 100 molar equivalents of SPDP, respectively) was determined using Ellman's reagent.

To prepare Pyd-labeled enzyme, a solution of thiolated enzyme (0.8 mg/ml in pH 6 buffer; 1 molar equivalent) was added to a solution (70 or 160 µl) of Pyd-SMCC (Example 16) in pH 6 buffer (20 and 40 equivalents, respectively), and the resultant mixture was allowed to react for 3 hr at 4°C under argon atmosphere in the dark. Following the reaction, a solution of N-ethyl maleimide (1 M) in methanol was added (final maleimide concentration, 50 mM), and the mixture was allowed to react for 1 hr. The mixture was then dialyzed exhaustively in 12-14,000 MW cut-off dialysis tubing against 10 mM PBS, pH 7, at 4°C in the dark. The ratio of G6PDH:Pyd (1:2 and 1:6, respectively) was calculated by measuring the absorbance at 280 and 326 nm.

### Example 18

### EMIT Assay

### A. Measurement of Catalytic Activity of G6PDH

In the following studies, G6PDH concentrations (unlabeled as well as various pyridinium-labeled forms) were diluted as necessary to achieve an enzyme concentration of 5 µg/ml in 50 mM Tris buffer, pH 7.5, prior addition to the microtitre plate wells. Into each well of a 96-well microtitre plate was placed 100 uL of Tris buffer and 50 µl of serially diluted enzyme (5 µg/ml or less). As a control, one row of wells was prepared without enzyme. To each of the wells was added 20 uL of a substrate solution (250 mM D-glucose-6-phosphate and 50 mM NAD⁺ in 50 mM Tris, pH 7.5) and the change in absorbance at 340 nm was recorded as a function of time, using a V-Max microplate reader (Molecular Devices Corp. Menlo Park, CA). Enzyme activities were typically recorded in units of mOD/minute.

### B. Effect of Antibody on Catalytic Activity of Pyridinium-Enzyme Conjugate

To the wells of a 96-well microtitre plate were added 100 µl of 50 mM Tris buffer, pH 7.5, and 50 µL of pyridinium-specific antibody, serially diluted across the plate. To each well was then added 100 µl of pyridinium-labeled enzyme to achieve an enzyme concentration effective to produce about 200-300 mOD/min of enzyme activity in the absence of antibody. After the plate had been allowed to incubate for a selected time (5-20 minutes) at room temperature, 50 µl of substrate solution (see above) was added, producing final concentrations of 50 mM G6P and 10 mM NAD⁺. The change in absorbance as a function of time was then measured at 340 nm, and the percent "down modulation" (decrease in catalytic activity) in the presence of various concentrations of antibody was determined relative to the activity observed in the absence of antibody (defined as 100% activity).

### C. EMIT Assay

To measure urinary levels of native free pyridinoline, a 50 µL aliquot of pyridinoline standard (0 to 3000 nM Pyd in 50 mM Tris, pH 7.5) or urine sample (diluted 1:5 in Tris, pH 7.5) was added to each well of a microtitre plate, followed by 100 uL of suitably diluted antibody (determined in section B above) in the same Tris buffer. After the plate had been incubated at room temperature for a selected time (2, 10, or 30 min), 100 µl of diluted EDC-coupled pyridinoline-G6PDH conjugate (effective to produce an activity of 200-300 mOD/minute in the absence of antibody) was added to each well, followed by incubation at room temperature for 5, 10, 15, or 30 minutes. Following the last incubation period, 60 uL of substrate solution (see above) was added to each well, and G6PDH activity was measured at 340 nm as above. The activities measured with the various Pyd standards were used to construct a Pyd calibration curve (e.g., see Fig. 10). This calibration curve was then used to determine the levels of Pyd in the urine samples, using the Softmax 2.22 software furnished with the Vₘₐₓ Microplate Reader (Molecular Devices Corp.).

### Example 19

### Preparation of Anti-Pyridinoline Antiserum

New Zealand white rabbits (a total of 59) for immunization were divided into eight groups according to the immunization protocol, as indicated below in Table 11. The immunization dose was 200 µg of H-Pyd-BSA (Example 3A), low-hapten H-Pyd-BSA immunogen (prepared as in Example 3A for H-Pyd-BSA, but with a lower Pyd:BSA stoichiometry), or H-Pyd-KLH (Example 3B), in 1.0 ml PBS mixed with 1.0 ml of Ribi adjuvant (Ribi ImmunoChemical Research, Inc.). Initial immunization was by subcutaneous injections at multiple sites, and subsequent booster immunizations were given at three week intervals intramuscularly. Antiserum was collected 10 days after each immunization.

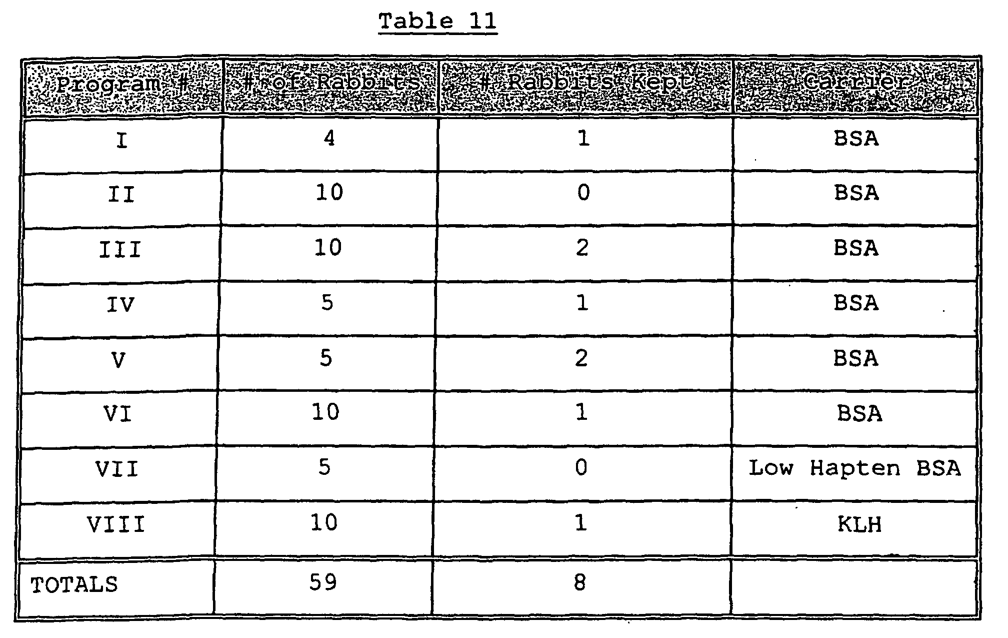

Upon collection, each antiserum was tested for Pyd binding affinity using the assay format described in Example 8. In brief, binding of anti-Pyd antibodies from the serum to Pyd immobilized on a solid support was detected using an alkaline phosphatase-labeled goat anti-rabbit IgG antibody reagent.

Immunized animals were kept if their antisera satisfied the following criteria, defined further in the following paragraph: AA < 20%, Pyd-peptide < 10%, titer > 5000, and a 0 to 25 nM Pyd signal separation of > 10% of total modulated signal.

Profiles of the most strongly reactive antisera are shown in Table 12 below, as measured using the assay format described in Example 8. The first column indicates the immunization program from which the rabbit antiserum came. The second column indicates the bleeds which were pooled for analysis. The column marked "titer" indicates the dilution of each antiserum necessary to achieve an optical density reading of 1.2 to 1.6 with a Pyd-negative sample (no Pyd present) in the immunoassay. The column marked "AA" shows the cross-reactivity of each antiserum with the amino acid mixture described in Example 10. The column marked "Pyd-pep >1000 MW" shows the cross-reactivity of each antiserum with Pyd-peptides (>1000 MW). The last column shows the separation between signals for 0 and 25 nM Pyd samples as a fraction of the total modulated signal.

**Table 12**

| **Rabbit #** | **Bleeds** | **Titer** | **AA** | **Pyd-pep. >1000 MW** | **Sens. 25 nM** |
|---|---|---|---|---|---|
| I-3 | 21-28 | 200K¹ | 2% | 4.6% | 18% |
| III-3 | 11-18 | 20K | 16% | 8.3% | 37% |
| III-5 | 11-18 | 52K | 1% | 8.1% | 13% |
| IV-4 | 4-14 | 84K | 4% | 4.9% | 10% |
| V-3 | 4-14 | 22K | 18% | 4.0% | 15% |
| V-4 | 11-14 | 9700 | 15% | 5.2% | 29% |
| VI-8 | 2-11 | 30K | 10% | 0.6% | 61% |
| VIII-4 | 3-10 | 34K | ∼0% | 3.4% | 11% |

| | | | | | |
|---|---|---|---|---|---|
| ¹K = × 1000. | | | | | |

As can be seen, rabbits III-3, V-4, and VI-8 showed significant modulation of signal from 0 to 25 nM N-Pyd. The serum with highest activity (VI-8) was selected for use in the N-Pyd assays described herein.

### Example 20

### Immunoassay Using Polyclonal Antibody Reagent

The following immunoassay was performed using rabbit polyclonal antibody VI-8 characterized in Tables 10 and 12 above, and the N-Pyd coated microtiter plate described in Example 8A.

N-Pyd standard solutions and urine samples were tested in duplicate. Urine samples were filtered through a nitrocellulose membrane by centrifugation (spin filtration) prior to assay.

Following the addition of sample or standard (25 µl/well), 125 µl/well of VI-8 antiserum diluted 20,000-fold in assay buffer was added, and the assay plate was incubated at 4°C overnight. After the plate was washed 3 times with 300 µl/well of wash buffer, 150 µl/well of goat anti-rabbit IgG-alkaline phosphatase conjugate (1:1000 dilution in assay buffer) was added, and the plate was incubated at room temperature for 1 h. The wells were then washed 3 times with wash buffer.

To each well was added 150 uL of enzyme substrate solution (2 mg/ml of p-nitrophenylphosphate (Sigma) in 1.0 M diethanolamine, pH 9.8, containing 1 mM MgCl₂). Following a 1 hour incubation at room temperature, 50 µl of 3.0 N NaOH was added to each well to stop the enzymatic reaction. The optical density at 405 nm was then measured with a Vmax reader (Molecular Devices Corp.).

The optical density readings (405 nm) from duplicate samples were averaged, and the averaged readings from the N-Pyd standards were used to construct a standard curve of OD reading vs. N-Pyd concentration. From this curve, the free N-Pyd crosslink concentration in each sample was determined.

## Claims

1. An antibody obtainable by immunizing an animal with hydrolyzed pyridinoline or hydrolyzed deoxypyridinoline which may be conjugated to a carrier, said antibody being **characterized by**:
immunospecific binding to pyridinium crosslinks selected from native free pyridinoline and/or native free deoxypyridinoline, and
a ratio of reactivity toward said selected native free pyridinium crosslink(s) and urinary pyridinium peptides larger than 1,000 daltons in molecular weight of greater than 3:1.

2. The antibody of Claim 1, which has a ratio of reactivity toward native free pyridinoline and native free deoxypyridinoline of greater than 5:1.

3. The antibody of Claim 2, obtained from a cell line identified by ATCC No. HB11089.

4. The antibody of Claim 1, which has a ratio of reactivity toward native free deoxypyridinoline and native free pyridinoline of greater than 25:1.

5. The antibody of Claim 1, which has a ratio of reactivity toward native free pyridinoline and native free deoxypyridinoline of between 2:1 and 1:2.

6. The antibody according to any preceding claim, wherein said antibody is a monoclonal antibody.

7. The antibody according to any of Claims 1, 2, 4 or 5, wherein said antibody is a polyclonal antibody.

8. A diagnostic kit for use in assaying a bone collagen degradation level in a human subject, comprising
an antibody according to any of claims 1 to 7, and
detection means for detecting the amount of immunocomplex formed by reaction of said antibody with such free pyridinium crosslinks.

9. The kit of claim 8, which further includes a solid-phase support having surface-attached free pyridinium crosslink molecules effective to compete with such free pyridinium crosslinks present in the sample for binding to said antibody, and said antibody is a reporter-labeled or reporter-labelable antibody.

10. The kit of claim 8, which further includes a solid-phase support to which the antibody is attached, and a reporter-labeled or reporter-labelable free pyridinium crosslink which is effective to compete with such free pyridinium crosslinks present in the sample, for binding to the antibody on the support.

11. The kit of claim 8, wherein the detection means includes a reporter enzyme effective to produce a colorimetric signal proportional to the amount of such an immunocomplex formed.

12. The kit of claim 8, wherein the detection means includes an enzyme which is labeled with the selected pyridinium crosslink; said labeled enzyme is effective to compete with such free pyridinium crosslinks present in the sample, for binding to the antibody, where binding of the antibody to the labeled enzyme causes a decrease in the catalytic activity of the labeled enzyme.

13. The kit according to any of claim 8 to 12, which further includes a filter membrane selected from the group consisting of nitrocellulose, Immobilon®-CD, and Immobilon®-PSQ, for sample filtration.

14. A method of assaying bone collagen degradation level in a human subject, comprising
reacting a non-hydrolized human urine sample with an antibody according to any of claims 1 to 7,
by said reacting, forming an immunoplex between the antibody and such selected pyridinium crosslinks present in the sample, and
from the amount of immunocomplex formed, determining the level of the selected pyridinium crosslinks in the sample, whereby a determined level which is above that characteristic of normal subjects indicates the presence of an elevated rate of bone collagen degradation level in the subject.

15. The method of claim 14, wherein said antibody has a ratio of reactivity toward native free pyridinoline and native free deoxypyridinoline of greater than 5:1.

16. The method of claim 14, wherein the level of native free pyridinoline measured is related to the concentration of total hydrolyzed pyridinoline in the sample by a linear relationship having a slope of 0.3-0.5.

17. The method of claim 14, wherein said antibody has a ratio of reactivity toward native free deoxypyridinoline and native free pyridinoline of greater than 25:1.

18. The method of claim 14, wherein said antibody has a ratio of reactivity toward native free pyridinoline and native free deoxypyridinoline of between 2:1 and 1:2.

19. The method of any of claims 14 to 18, wherein said reacting for competing with such selected pyridinium crosslinks in a sample includes contacting the urine sample with a solid-phase support having surface-attached peptide-free pyridinoline or deoxypyridinoline.

20. The method of any of claims 14 to 18, wherein said reacting includes contacting the urine sample with a solid phase support having a surface-attached antibody in accordance with claim 1.

21. The method according to any of claims 14 to 20, wherein prior to said reacting, the sample is filtered through a filter membrane selected from the group consisting of nitrocellulose, Immobilon®-CD, and Immobilon®-PSQ, where the membrane is effective to remove a substance that bind non-specifically to antibodies, but allows substantially all of the native free pyridinium crosslinks to the sample to pass through.

22. A method of preparing antibodies in accordance with any one of claims 1 to 7, the method comprising
immunizing an animal with a pyridinium crosslink selected from hydrolyzed pyridinoline and hydrolyzed deoxypyridinoline,
obtaining antibodies from the animal, and
selecting antibodies which have a binding affinity of greater than 5x10⁷/molar for native, non-glycosylated, peptide-free pyridinoline or native, peptide-free deoxypyridinoline.

23. The method of claim 22, wherein the selected pyridinium crosslink is native, non-glycosylated, peptide-free pyridinoline.

24. The method of claim 22, wherein the selected pyridinium crosslink is native, peptide-free deoxypyridinoline.

25. The method of claim 22, wherein the animal is a rabbit or a mouse.

## Patentansprüche

1. Antikörper, erhältlich durch Immunisieren eines Tieres mit hydrolysiertem Pyridinolin oder hydrolysiertem Desoxypyridinolin, welche an einen Träger gebunden sein können, wobei der Antikörper durch
das immunspezifische Binden an Pyridinium-Vernetzungen, ausgewählt aus nativem, freiem Pyridinolin und/oder nativem, freiem Desoxypyridinolin, und
ein Reaktivitätsverhältnis gegenüber der (den) ausgewählten nativen, freien Pyridinium-Vernetzung(en) und den Harn-Pyridiniumpeptiden mit einem Molekulargewicht von mehr als 1.000 Dalton von größer als 3:1
**gekennzeichnet** ist.

2. Antikörper nach Anspruch 1, welcher ein Reaktivitätsverhältnis gegenüber nativem, freiem Pyridinolin und nativem, freiem Desoxypyridinolin von größer als 5:1 aufweist.

3. Antikörper nach Anspruch 2, erhalten aus einer Zelllinie, welche durch die ATCC Nr. HB11089 identifiziert ist.

4. Antikörper nach Anspruch 1, welcher ein Reaktivitätsverhältnis gegenüber nativem, freiem Desoxypyridinolin und nativem, freiem Pyridinolin von größer als 25:1 aufweist.

5. Antikörper nach Anspruch 1, welcher ein Reaktivitätsverhältnis gegenüber nativem, freiem Pyridinolin und nativem, freiem Desoxypyridinolin zwischen 2:1 und 1:2 aufweist.

6. Antikörper nach einem vorhergehenden Anspruch, wobei der Antikörper ein monoklonaler Antikörper ist.

7. Antikörper nach einem der Ansprüche 1, 2, 4 oder 5, wobei der Antikörper ein polyklonaler Antikörper ist.

8. Diagnostischer Kit zur Verwendung bei der Untersuchung der Abnahme des Knochenkollagenspiegels in einem menschlichen Lebewesen, umfassend
einen Antikörper nach einem der Ansprüche 1 bis 7 und ein Nachweismittel zum Nachweisen der Menge des Immunkomplexes, welcher durch die Reaktion des Antikörpers mit solchen freien Pyridinium-Vernetzungen gebildet wird.

9. Kit nach Anspruch 8, welcher weiter einen Festphasenträger mit oberflächengebundenen freien vernetzten Pyridiniummolekülen einschließt, die wirksam sind, mit solchen freien Pyridinium-Vernetzungen, die in der Probe vorliegen, zur Bindung an den Antikörper zu konkurrieren, und wobei der Antikörper ein Reporter-markierter oder Reportermarkierbarer Antikörper ist.

10. Kit nach Anspruch 8, welcher weiter einen Festphasenträger, an welchen der Antikörper gebunden ist, und eine Reporter-markierte oder Reportermarkierbare freie Pyridinium-Vernetzung einschließt, welche wirksam ist, mit solchen freien Pyridinium-Vernetzungen, die in der Probe vorliegen, zur Bindung an den Antikörper auf dem Träger zu konkurrieren.

11. Kit nach Anspruch 8, wobei das Nachweismittel ein Reporterenzym einschließt, das wirksam ist, ein kolorimetrisches Signal proportional zu der Menge eines solchen gebildeten Immunkomplexes zu erzeugen.

12. Kit nach Anspruch 8, wobei das Nachweismittel ein Enzym einschließt, welches mit der ausgewählten Pyridinium-Vernetzung markiert ist, wobei das markierte Enzym wirksam ist, mit solchen freien Pyridinium-Vernetzungen, die in der Probe vorliegen, zur Bindung an den Antikörper zu konkurrieren, wobei das Binden des Antikörpers an das markierte Enzym eine Abnahme in der katalytischen Aktivität des markierten Enzymes bewirkt.

13. Kit nach einem der Ansprüche 8 bis 12, welcher weiter eine Filtermembran, ausgewählt aus der Gruppe, bestehend aus Nitrozellulose, Immobilon®-CD, und Immobilon®-PSQ, für die Probenfiltration einschließt.

14. Verfahren zur Untersuchung der Abnahme des Knochenkollagenspiegels in einem menschlichen Patienten, umfassend
das Reagieren einer nicht hydrolysierten, menschlichen Urinprobe mit einem Antikörper nach einem der Ansprüche 1 bis 7,
wobei durch das Reagieren ein Immunkomplex zwischen dem Antikörper und solchen ausgewählten Pyridinium-Vernetzungen, die in der Probe vorliegen, gebildet wird, und
aus der Menge des gebildeten Immunkomplexes der Spiegel der ausgewählten Pyridinium-Vernetzungen in der Probe bestimmt wird, wobei ein bestimmter Spiegel, welcher über dem für einen normalen Patienten Charakteristischen ist, die Anwesenheit einer erhöhten Geschwindigkeit der Abnahme des Knochenkollagenspiegels in dem Patienten anzeigt.

15. Verfahren nach Anspruch 14, wobei der Antikörper ein Reaktivitätsverhältnis gegenüber nativem, freiem Pyridinolin und nativem freiem Desoxypyridinolin von größer als 5:1 aufweist.

16. Verfahren nach Anspruch 14, wobei der gemessene Spiegel von nativem, freiem Pyridinolin auf die Konzentration des gesamten hydrolysierten Pyridinolins in der Probe über eine lineare Beziehung mit einer Steigung von 0,3 - 0,5 bezogen wird.

17. Verfahren nach Anspruch 14, wobei der Antikörper ein Reaktivitätsverhältnis gegenüber freiem, nativem Desoxypyridinolin und nativem, freiem Pyridinolin von größer als 25:1 aufweist.

18. Verfahren nach Anspruch 14, wobei der Antikörper ein Reaktivitätsverhältnis gegenüber nativem, freiem Pyridinolin und nativem, freiem Desoxypyridinolin von zwischen 2:1 und 1:2 aufweist.

19. Verfahren nach einem der Ansprüche 14 bis 18, wobei das Reagieren zum Konkurrieren mit solchen ausgewählten Pyridinium-Vernetzungen in einer Probe das Kontaktieren der Urinprobe mit einem Festphasenträger mit oberflächengebundenem, peptidfreiem Pyridinolin oder Desoxypyridinolin einschließt.

20. Verfahren nach einem der Ansprüche 14 bis 18, wobei das Reagieren das Kontaktieren der Urinprobe mit einem Festphasenträger mit einem oberflächengebundenen Antikörper gemäß Anspruch 1 einschließt.

21. Verfahren nach einem der Ansprüche 14 bis 20, wobei vor dem Reagieren die Probe durch eine Filtermembran, ausgewählt, aus der Gruppe bestehend aus Nitrozellulose, Immobilon®-CD und Immobilon®-PSQ, filtriert wird, wobei die Membran wirksam ist, eine Substanz zu entfernen, die unspezifisch an Antikörper bindet, aber im wesentlichen allen nativen freien Pyridinium-Vernetzungen gestattet mit der Probe durchzuströmen.

22. Verfahren zum Herstellen von Antikörpern gemäß einem der Ansprüche 1 bis 7, wobei das Verfahren
das Immunisieren eines Tieres mit einer Pyridinium-Vernetzung, ausgewählt aus hydrolysiertem Pyridinolin und hydrolysiertem Desoxypyridinolin,
das Erhalten der Antikörper aus dem Tier, und
das Auswählen der Antikörper, welche eine Bindungsaffinität von größer als 5x10⁷/molar für natives, nicht-glycosyliertes, peptidfreies Pyridinolin oder natives, peptidfreies Desoxypyridinolin aufweisen,
umfaßt.

23. Verfahren nach Anspruch 22, wobei die ausgewählte Pyridinolin-Vernetzung natives, nicht-glycosyliertes, peptidfreies Pyridinolin ist.

24. Verfahren nach Anspruch 22, wobei die ausgewählte Pyridinium-Vernetzung natives, peptidfreies Desoxypyridinolin ist.

25. Verfahren nach Anspruch 22, wobei das Tier ein Kaninchen oder eine Maus ist.

## Revendications

1. Anticorps susceptible d'être obtenu en immunisant un animal par de la pyridinoline hydrolysée ou de la désoxypyridinoline hydrolysée, qui peut être conjugué à un support, ledit anticorps étant **caractérisé par** :
la liaison immunospécifique à des réticulations de pyridinium choisies parmi les pyridinolines exemptes de composé natif et/ou les désoxypyridinolines exemptes de composé natif, et
un rapport de réactivité vis-à-vis de ladite ou desdites réticulation(s) de pyridinium exemptes de composé natif et des peptides de pyridinium urinaires de masse moléculaire supérieure à 1000 daltons, supérieur à 3 : 1.

2. Anticorps selon la revendication 1, qui présente un rapport de réactivité vis-à-vis de la pyridinoline exempte de composé natif et de la désoxypyridinoline exempte de composé natif, supérieur à 5 : 1.

3. Anticorps selon la revendication 2, obtenu à partir d'une lignée cellulaire identifiée par ATCC No. HB11089.

4. Anticorps selon la revendication 1, qui présente un rapport de réactivité vis-à-vis de la désoxypyridinoline exempte de composé natif et de la pyridinoline exempte de composé natif, supérieur à 25 : 1.

5. Anticorps selon la revendication 1, qui présente un rapport de réactivité vis-à-vis de la pyridinoline exempte de composé natif et de la désoxypyridinoline exempte de composé natif, entre 2 : 1 et 1 : 2.

6. Anticorps selon l'une des revendications précédentes, dans lequel ledit anticorps est un anticorps monoclonal.

7. Anticorps selon l'une quelconque des revendications 1, 2, 4 ou 5, dans lequel ledit anticorps est un anticorps polyclonal.

8. Trousse de diagnostic pour être utilisée dans l'analyse du taux de dégradation de collagène osseux chez un sujet humain, comprenant
un anticorps selon l'une quelconque des revendications 1 à 7, et des moyens de détection pour détecter la quantité d'immunocomplexe formé par réaction dudit anticorps avec de telles réticulations exemptes de pyridinium.

9. Trousse selon la revendication 8, qui comprend en outre un support en phase solide ayant des molécules de réticulation exemptes de pyridinium fixées à la surface, efficaces pour entrer en compétition avec de telles réticulations exemptes de pyridinium présentes dans l'échantillon pour la liaison audit anticorps, et ledit anticorps est un anticorps marqué par un marqueur ou un anticorps susceptible d'être marqué par un marqueur.

10. Trousse selon la revendication 8, qui comprend en outre un support en phase solide auquel l'anticorps est fixé, et une réticulation exempte de pyridinium, marquée par un marqueur ou susceptible d'être marquée par un marqueur, qui est efficace pour entrer en compétition avec de telles réticulations exemptes de pyridinium présentes dans l'échantillon, pour la liaison de l'anticorps au support.

11. Trousse selon la revendication 8, dans laquelle le moyen de détection comprend une enzyme de marquage efficace pour produire un signal calorimétrique proportionnel à la quantité de cet immunocomplexe formé.

12. Trousse selon la revendication 8, dans laquelle le moyen de détection comprend une enzyme qui est marquée par la réticulation de pyridinium choisie ; ladite enzyme marquée étant efficace pour entrer en compétition avec de telles réticulations exemptes de pyridinium présentes dans l'échantillon, pour la liaison à l'anticorps, la liaison de l'anticorps à l'enzyme marquée provoquant une diminution de l'activité catalytique de l'enzyme marquée.

13. Trousse selon l'une quelconque des revendications 8 à 12, qui comprend en outre une membrane filtre choisie dans le groupe constitué par la nitrocellulose, Immobilon®-CD et Immobilon®PCB pour la filtration de l'échantillon.

14. Procédé pour analyser le niveau de dégradation du collagène osseux chez un sujet humain, comprenant les étapes consistant à
faire réagir un échantillon d'urine humaine non hydrolysée avec un anticorps selon l'une quelconque des revendications 1 à 7,
former par ladite réaction un immunocomplexe entre l'anticorps et les réticulations de pyridinium choisies présentes dans l'échantillon, et
à partir de la quantité d'immunocomplexe formé, déterminer dans l'échantillon la quantité de réticulations de pyridinium choisies, où une quantité déterminée, qui est supérieure à la caractéristique des sujets normaux, indique un taux élevé de niveau de dégradation de collagène osseux chez le sujet.

15. Procédé selon la revendication 14, dans lequel ledit anticorps présente un rapport de réactivité vis-à-vis de la pyridinoline exempte de composé natif et de la désoxypyridinoline exempte de composé natif supérieur à 5 : 1.

16. Procédé selon la revendication 14, dans lequel la quantité de pyridinoline exempte de composé natif mesurée est reliée à la concentration de la pyridinoline hydrolysée totale dans l'échantillon par une relation linéaire ayant une pente de 0,3 - 0,5.

17. Procédé selon la revendication 14, dans lequel ledit anticorps présente un rapport de réactivité vis-à-vis de la désoxypyridinoline exempte de composé natif et de la pyridinoline exempte de composé natif supérieur à 25 : 1.

18. Procédé selon la revendication 14, dans lequel ledit anticorps présente un rapport de réactivité vis-à-vis de la pyridinoline exempte de composé natif et de la désoxypyridinoline exempte de composé natif entre 2 : 1 et 1 : 2.

19. Procédé selon l'une quelconque des revendications 14 à 18, dans lequel ladite réaction de compétition avec les réticulations de pyridinium choisies dans un échantillon comprend la mise en contact de l'échantillon d'urine avec un support en phase solide ayant une pyridinoline ou une désoxypyridinoline exempte de peptide fixée en surface.

20. Procédé selon l'une quelconque des revendications 14 à 18, dans lequel ladite réaction comprend la mise en contact de l'échantillon d'urine avec un support en phase solide ayant un anticorps fixé en surface selon la revendication 1.

21. Procédé selon l'une quelconque des revendications 14 à 20, dans lequel avant ladite réaction, on filtre l'échantillon à travers une membrane de filtre choisie dans le groupe constitué par le nitrocellulose, Immobilon®-CD et Immobilon®PCB, où la membrane est efficace pour éliminer une substance qui se lie non spécifiquement aux anticorps, mais permet que sensiblement la totalité du pyridinium exempt de produit natif se réticule à l'échantillon pour passer à travers.

22. Procédé de préparation d'anticorps selon l'une quelconque des revendications 1 à 7, le procédé comprenant les étapes consistant à
immuniser un animal par une réticulation de pyridinium choisie parmi la pyridinoline hydrolysée et la désoxypyrodinoline hydrolysée,
obtenir des anticorps à partir de l'animal, et
choisir des anticorps qui ont une affinité de liaison supérieure à 5 x 10⁷ /molaire pour la pyridinoline exempte de peptide, non glycosylée, native ou de désoxypyridinoline exempte de peptide, native.

23. Procédé selon la revendication 22, dans lequel la réticulation pyridinium choisie est une pyridinoline exempte de peptide, non glycosylée, native.

24. Procédé selon la revendication 22, dans lequel la réticulation de pyridinium choisie est une désoxypyridinoline exempte de peptide, native.

25. Procédé selon la revendication 22, dans lequel l'animal est un lapin ou une souris.
